# EUROPÄISCHE PATENTSCHRIFT

(11) **EP 0 759 908 B1**
(45) Veröffentlichungstag und Bekanntmachung des Hinweises auf die Patenterteilung: **02.04.2003**
(21) Anmeldenummer: 95919398.8
(22) Anmeldetag: 05.05.1995
(51) Int. Cl.: C07D 239/22, C07C 255/50, C07C 211/52, C07C 265/12, A01N 43/54

(54) **SUBSTITUIERTE DIAZACYCLOHEXANDI(THI)ONE**
SUBSTITUTED DIAZACYCLOHEXANDI(THI)ONES
DIAZACYCLOHEXANDI(THI)ONES SUBSTITUEES

(30) Priorität: 18.05.1994 DE 4417352; 04.01.1995 DE 19500118
(43) Veröffentlichungstag der Anmeldung: 05.03.1997
(73) Patentinhaber: Bayer CropScience AG, 40789 Monheim (DE)
(72) Erfinder: DREWES, Mark-Wilhelm, D-40764 Langenfeld (DE); ANDREE, Roland, D-40764 Langenfeld (DE); SCHALLNER, Otto, D-40789 Monheim (DE); SANTEL, Hans-Joachim, D-51371 Leverkusen (DE); DOLLINGER, Markus, D-51381 Leverkusen (DE)
(86) Internationale Anmeldenummer: EP9501700
(87) Internationale Veröffentlichungsnummer: WO95031440

(56) Entgegenhaltungen:
- EP-A- 0 162 669
- DE-A- 4 206 416
- US-A- 4 927 451
- CHEMICAL ABSTRACTS, vol. 121, no. 11, 1994, Columbus, Ohio, US; abstract no. 127858h, Seite 389 ; in der Anmeldung erwähnt & JP,A,0 692 943 (NISSAN) 5. April 1994 in der Anmeldung erwähnt

## Beschreibung

Die Erfindung betrifft neue substituierte Diazacyclohexandi(thi)one, ein Verfahren zu ihrer Herstellung und ihre Verwendung als Herbizide.

Es ist bekannt, dass bestimmte Diazacyclohexandi(thi)one herbizide Eigenschaften aufweisen (vgl. US 4927451 und JP 06092943). Die aus den angegebenen Patent-Publikationen bekannten Verbindungen haben jedoch keine nennenswerte Bedeutung erlangt.

Es wurden nun die neuen Diazacyclohexandi(thi)one der allgemeinen Formel (I) gefunden, 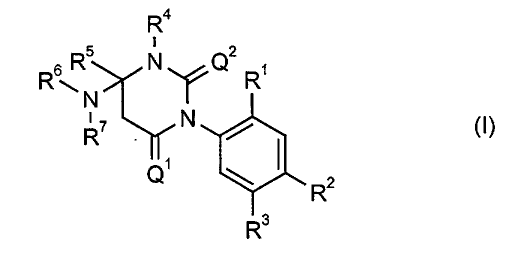 in welcher
- Q¹: für Sauerstoff oder Schwefel steht,
- Q²: für Sauerstoff oder Schwefel steht,
- R¹: für Wasserstoff, Cyano, Nitro, Fluor, Chlor, Brom oder für gegebenenfalls durch Fluor und/oder Chlor substituiertes Alkyl oder Alkoxy mit jeweils 1 bis 4 Kohlenstoffatomen steht,
- R²: für Wasserstoff, Cyano, Nitro, Thiocarbamoyl, Fluor, Chlor, Brom oder für jeweils gegebenenfalls durch Fluor und/oder Chlor substituiertes Alkyl oder Alkoxy mit jeweils 1 bis 4 Kohlenstoffatomen steht,
- R³: für die nachstehende Gruppierung steht,

-A¹-A²-A³

in welcher
- A¹: für eine Einfachbindung, für Sauerstoff, Schwefel, -SO-, -SO₂-, -CO- oder die Gruppierung -N-A⁴- steht, worin A⁴ für Wasserstoff, Hydroxy, C₁-C₄-Alkyl, C₁-C₄-Alkoxy, Phenyl, C₁-C₄-Alkylsulfonyl oder Phenylsulfonyl steht,
- A¹: weiterhin für jeweils gegebenenfalls durch Fluor, Chlor oder Brom substituiertes C₁-C₆-Alkandiyl, C₂-C₆-Alkendiyl, C₂-C₆-Azaalkendiyl, C₂-C₆-Alkindiyl, C₃-C₆-Cycloalkandiyl, C₃-C₆-Cycloalkendiyl oder Phenylen steht,
- A²: für eine Einfachbindung, für Sauerstoff, Schwefel, -SO-, -SO₂-, -CO- oder die Gruppierung -N-A⁴- steht, worin A⁴ für Wasserstoff, Hydroxy, C₁-C₄-Alkyl, C₁-C₄-Alkoxy, Phenyl, C₁-C₄-Alkylsulfonyl oder Phenylsulfonyl steht,
- A²: weiterhin für jeweils gegebenenfalls durch Fluor, Chlor oder Brom substituiertes C₁-C₆-Alkandiyl, C₂-C₆-Alkendiyl, C₂-C₆-Azaalkendiyl C₂-C₆-Alkindiyl, C₃-C₆-Cycloalkandiyl, C₃-C₆-Cycloalkendiyl oder phenylen steht,
- A³: für Wasserstoff, Hydroxy, Mercapto, Amino, Cyano, Isocyano, Thiocyanato, Nitro, Carboxy, Carbamoyl, Thiocarbamoyl, Sulfo, Chlorsulfonyl, Halogen, für jeweils gegebenenfalls durch Halogen oder C₁-C₄-Alkoxy substituiertes Alkyl, Alkoxy, Alkylthio, Alkylsulfinyl, Alkylsulfonyl, Alkylamino, Dialkylamino, Alkoxycarbonyl oder Dialkoxy(thio)phosphoryl mit jeweils 1 bis 6 Kohlenstoffatomen in den Alkylgruppen, für jeweils gegebenenfalls durch Halogen substituiertes Alkenyl, Alkenyloxy, Alkenylamino, Alkylidenamino, Alkenyloxycarbonyl, Alkinyl, Alkinyloxy, Alkinylamino oder Alkinyloxycarbonyl mit jeweils 2 bis 6 Kohlenstoffatomen in den Alkenyl-, Alkyliden- oder Alkinylgruppen, für jeweils gegebenenfalls durch Halogen, Cyano, Carboxy, C₁-C₄-Alkyl und/oder C₁-C₄-Alkoxy-carbonyl substituiertes Cycloalkyl, Cycloalkyloxy, Cycloalkylalkyl, Cycloalkylalkoxy Cycloalkylidenamino, Cycloalkyloxycarbonyl oder Cycloalkylalkoxycarbonyl mit jeweils 3 bis 6 Kohlenstoffatomen in den Cycloalkylgruppen und gegebenenfalls 1 bis 4 Kohlenstoffatomen in den Alkylgruppen, oder für jeweils gegebenenfalls durch Nitro, Cyano, Carboxy, Halogen C₁-C₄-Alkyl, C₁-C₄-Halogenalkyl, C₁-C₄-Alkyloxy, C₁-C₄-Halogenalkyloxy und/oder C₁-C₄-Alkoxy-carbonyl substituiertes Phenyl, Phenyloxy, Phenyl-C₁-C₄-alkyl, Phenyl-C₁-C₄-alkoxy, Phenyloxycarbonyl oder Phenyl-C₁-C₄-alkoxycarbonyl, Oxetanyl, (jeweils gegebenenfalls ganz oder teilweise hydriertes) Pyrrolyl, Pyrazolyl, Imidazolyl, Triazolyl, Furyl, Thienyl, Oxazolyl, Isoxazolyl, Thiazolyl, Isothiazolyl, Oxadiazolyl, Thiadiazolyl, Pyridinyl, Pyrimidinyl, Triazinyl, Pyrazolyl-C₁-C₄-alkyl, Furyl-C₁-C₄-alkyl, Thienyl-C₁-C₄-alkyl, Oxazolyl-C₁-C₄-alkyl, Isoxazol-C₁-C₄-alkyl, Thiazol-C₁-C₄-alkyl, Pyridinyl-C₁-C₄-alkyl, Pyrimidinyl-C₁-C₄-alkyl, Pyrazolylmethoxy, Furylmethoxy, für Perhydropyranylmethoxy oder Pyridylmethoxy steht,
oder die Reste R² und R³ zusammen für eine der nachstehenden Gruppierungen stehen
-Q³-CQ⁴-Q⁵-, -Q³-C(R⁸,R⁹)-Q⁵-, -C(R⁸,R⁹)-Q³-CQ⁴-,
-Q³-C(R⁸,R⁹)-C(R⁸,R⁹)-, -Q³-C(R⁸,R⁹)-C(R⁸,R⁹)-Q⁵-,
-C(R⁸,R⁹)-C(R⁸,R⁹)-CQ⁴-, -Q³-C(R⁸)=C(R⁸)-, -C(R⁸)=C(R⁸)-CQ⁴-,
-Q³-C(R⁸,R⁹)-CQ⁴-, -N(R¹⁰)-C(R⁸;R⁹)-CQ⁴-, -Q³-CQ⁴-C(R⁸,R⁹)-,
-Q³-CQ⁴-N(R¹⁰)-, -Q³-C(R⁸,R⁹)-CQ⁴-N(R¹⁰)-,
-C(R⁸,R⁹)-Q³-CQ⁴-N(R¹⁰)-, -C(R⁸,R⁹)-C(R⁸,R⁹)-N(R¹⁰)-,
-C(R⁸,R⁹)-C(R⁸,R⁹)-CQ⁴-N(R¹⁰)-, -C(R⁸)=C(R⁸)-N(R¹⁰)-,
-C(R⁸)=C(R⁸)-CQ⁴-N(R¹⁰)-, -C(R⁸,R⁹)-CQ⁴-N(R¹⁰)-,
-N(R¹⁰)-C(R⁸;R⁹)-CQ⁴-N(R¹⁰)-, -C(R⁸)=N-N(R¹⁰)-,
-Q³-CQ⁴-C(R⁸,R⁹)-N(R¹⁰)-,
wobei
Q³, Q⁴ und Q⁵ gleich oder verschieden sind und jeweils für Sauerstoff oder Schwefel stehen,
R⁸ und R⁹ gleich oder verschieden sind und einzeln für Wasserstoff, Fluor, Chlor, Brom oder C₁-C₄-Alkyl stehen oder zusammen für C₂-C₅-Alkandiyl stehen, und
R¹⁰ für Wasserstoff, Hydroxy, für gegebenenfalls durch Cyano, Fluor, Chlor, C₁-C₄-Alkoxy, C₁-C₄-Alkyl-carbonyl oder C₁-C₄-Alkoxycarbonyl substituiertes Alkyl, Alkylcarbonyl, Alkoxycarbonyl oder Alkylsulfonyl mit jeweils 1 bis 6 Kohlenstoffatomen in den Alkylgruppen, für jeweils gegebenenfalls durch Fluor, Chlor oder Brom substituiertes Alkenyl oder Alkinyl mit jeweils 2 bis 6 Kohlenstoffatomen, für jeweils gegebenenfalls durch Fluor, Chlor, Brom oder C₁-C₄-Alkyl substituiertes Cycloalkyl oder Cycloalkylalkyl mit jeweils 3 bis 6 Kohlenstoffatomen in den Cycloalkylgruppen und gegebenenfalls 1 bis 3 Atomen in der Alkylgruppe, für jeweils gegebenenfalls durch Fluor und/oder Chlor substituiertes Alkoxy oder Alkenyloxy mit jeweils bis zu 6 Kohlenstoffatomen, oder für jeweils gegebenenfalls durch Cyano, Fluor, Chlor, C₁-C₄-Alkyl, C₁-C₄-Halogenalkyl, C₁-C₄-Alkoxy oder C₁-C₄-Halogenalkoxy substituiertes Benzyl oder Benzyloxy steht,
R⁴ für Amino, für gegebenenfalls durch Fluor und/oder Chlor substituiertes Alkyl mit 1 bis 4 Kohlenstoffatomen oder für Cycloalkyl mit 3 bis 6 Kohlenstoffatomen steht,
R⁵ für Wasserstoff oder für gegebenenfalls durch Fluor und/oder Chlor substituiertes Alkyl mit 1 bis 4 Kohlenstoffatomen steht,
R⁶ für Wasserstoff oder Alkyl mit 1 bis 4 Kohlenstoffatomen steht, und
R⁷ für Wasserstoff oder für Alkyl, Alkylcarbonyl, Alkoxycarbonyl, Alkylaminocarbonyl, Dialkylaminocarbonyl oder Alkylsulfonyl mit jeweils 1 bis 4 Kohlenstoffatomen in den Alkylgruppen steht, oder
R⁶ und R⁷ zusammen für Alkandiyl mit 2 bis 6 Kohlenstoffatomen oder eine Alkylidenimino-, Cycloalkylalkylidenimino- oder Arylalkylidenimino-Gruppierung mit jeweils bis zu 6 Kohlenstoffatomen im Alkylidenteil und gegebenenfalls 5 oder 6 Kohlenstoffatomen im Cycloalkylteil oder 6 oder 10 Kohlenstoffatomen im Arylteil stehen.

Gegenstand der Erfindung sind insbesondere Verbindungen der Formel (I), in welcher
- Q¹: für Sauerstoff steht,
- Q²: für Sauerstoff steht,
- R¹: für Wasserstoff, Fluor oder Chlor steht,
- R²: für Cyano, Chlor, Brom, Methyl oder Trifluormethyl steht,
- R³: für die nachstehende Gruppierung steht,

-A¹-A²-A³

in welcher
A¹ für eine Einfachbindung, für Sauerstoff, Schwefel, -SO-, -SO₂-, -CO- oder die Gruppierung -N-A⁴- steht, worin A⁴ für Wasserstoff, Hydroxy, Methyl, Ethyl, n- oder i-Propyl, Methoxy, Ethoxy, n- oder i-Propoxy, Methylsulfonyl oder Ethylsulfonyl steht,
A¹ weiterhin für Methylen, Ethan-1,1-diyl, Ethan-1,2-diyl, Propan-1,1-diyl, Propan-1,2-diyl, Propan-1,3-diyl, Ethen-1,2-diyl, Propen-1,2-diyl, Propen-1,3-diyl, Ethin-1,2-diyl, Propin-1,2-diyl oder Propin-1,3-diyl steht,
A² für eine Einfachbindung, für Sauerstoff, Schwefel, -SO-, -SO₂-, -CO- oder die Gruppierung -N-A⁴- steht, worin A⁴ für Wasserstoff, Hydroxy, Methyl, Ethyl, n- oder i-Propyl, Methoxy, Ethoxy, n- oder i-Propoxy, Methylsulfonyl, Ethylsulfonyl, n- oder i-Propylsulfonyl oder Phenylsulfonyl steht,
A² weiterhin für Methylen, Ethan-1,1-diyl, Ethan-1,2-diyl, Propan-1,1-diyl, Propan-1,2-diyl, Propan-1,3-diyl, Ethen-1,2-diyl, Propen-1,2-diyl, Propen-1,3-diyl, Ethin-1,2-diyl, Propin-1,2-diyl oder Propin-1,3-diyl steht,
A³ für Wasserstoff, Hydroxy, Amino, Cyano, Nitro, Carboxy, Carbamoyl, Sulfo, Fluor, Chlor, Brom, für jeweils gegebenenfalls durch Fluor, Chlor, Methoxy oder Ethoxy substituiertes Methyl, Ethyl, n- oder i-Propyl, n-, i-, s- oder t-Butyl, n-, i-, s- oder t-Pentyl, Methoxy, Ethoxy, n- oder i-Propoxy, n-, i-, s- oder t-Butoxy, n-, i-, s- oder t-Pentyloxy, Methylthio, Ethylthio, n- oder i-Propylthio, n-, i-, s- oder t-Butylthio, Methylsulfinyl, Ethylsulfinyl, n- oder i-Propylsulfinyl, Methylsulfonyl, Ethylsulfonyl, n- oder i-Propylsulfonyl, Methylamino, Ethylamino, n-oder i-Propylamino, n-, i-, s- oder t-Butylamino, Dimethylamino, Diethylamino, Methoxycarbonyl, Ethoxycarbonyl, n- oder i-Propoxycarbonyl, Dimethoxyphosphoryl, Diethoxyphosphoryl oder Dipropoxyphosphoryl, Diisopropoxyphosphoryl, für jeweils gegebenenfalls durch Fluor oder Chlor substituiertes Propenyl, Butenyl, Propenyloxy, Butenyloxy, Propenylamino, Butenylamino, Propylidenamino, Butylidenamino, Propenyloxycarbonyl, Butenyloxycarbonyl, Propinyl, Butinyl, Propinyloxy, Butinyloxy, Propinylamino, Butinylamino, Propinyloxycarbonyl oder Butinyloxycarbonyl, für jeweils gegebenenfalls durch Fluor, Chlor, Cyano, Carboxy, Methyl, Ethyl, n- oder i-Propyl, Methoxycarbonyl oder Ethoxycarbonyl substituiertes Cyclopropyl, Cyclobutyl, Cyclopentyl, Cyclohexyl, Cyclopropyloxy, Cyclobutyloxy, Cyclopentyloxy, Cyclohexyloxy, Cyclopropylmethyl, Cyclobutylmethyl, Cyclopentylmethyl, Cyclohexylmethyl, Cyclo-propylmethoxy, Cyclobutylmethoxy, Cyclopentylmethoxy, Cyclohexylmethoxy, Cyclopentylidenamino, Cyclohexylidenamino, Cyclopentyloxycarbonyl, Cyclohexyloxycarbonyl, Cyclopentylmethoxycarbonyl oder Cyclohexylmethoxycarbonyl, oder für jeweils gegebenenfalls durch Nitro, Cyano, Carboxy, Fluor, Chlor, Brom, Methyl, Ethyl, n- oder i-Propyl, Trifluormethyl, Methoxy, Ethoxy, n- oder i-Propoxy, Difluormethoxy, Trifluormethoxy, Methoxycarbonyl und/oder Ethoxycarbonyl substituiertes Phenyl, Phenyloxy, Benzyl, Phenylethyl, Benzyloxy, Phenyloxycarbonyl, Benzyloxycarbonyl, Oxetanyl, (jeweils gegebenenfalls ganz oder teilweise hydriertes) Pyrrolyl, Pyrazolyl, Imidazolyl, Triazolyl, Furyl, Thienyl, Oxazolyl, Isoxazolyl, Thiazolyl, Isothiazolyl, Oxadiazolyl, Thiadiazolyl, Pyridinyl, Pyrimidinyl, Triazinyl, Pyrazolylmethyl, Furylmethyl, Thienylmethyl, Oxazolylmethyl, Isoxazolmethyl, Thiazolmethyl, Pyridinyhnethyl, Pyrimidinylmethyl, Pyrazolylmethoxy, Furylmethoxy oder Pyridylmethoxy steht, oder die Reste R² und R³ zusammen für eine der nachstehenden Gruppierungen stehen
-Q³-CQ⁴-Q⁵-, -Q³-C(R⁸,R⁹)-Q⁵-, -C(R⁸,R⁹)-Q³-CQ⁴-,
-Q³-C(R⁸,R⁹)-C(R⁸,R⁹)-, -Q³-C(R⁸,R⁹)-C(R⁸,R⁹)-Q⁵-,
-C(R⁸,R⁹)-C(R⁸,R⁹)-CQ⁴-, -Q³-C(R⁸)=C(R⁸)-, -C(R⁸)=C(R⁸)-CQ⁴-,
-Q³-C(R⁸,R⁹)-CQ⁴-, -N(R¹⁰)-C(R⁸;R⁹)-CQ⁴-, -Q³-CQ⁴-C(R⁸,R⁹)-,
-Q³-CQ⁴-N(R¹⁰)-, -Q³-C(R⁸,R⁹)-CQ⁴-N(R¹⁰)-,
-C(R⁸,R⁹)-Q³-CQ⁴-N(R¹⁰)-, -C(R⁸,R⁹)-C(R⁸,R⁹)-N(R¹⁰)-,
-C(R⁸,R⁹)-C(R⁸,R⁹)-CQ⁴-N(R¹⁰)-, -C(R⁸)=C(R⁸)-N(R¹⁰)-,
-C(R⁸)=C(R⁸)-CQ⁴-N(R¹⁰)-, -C(R⁸,R⁹)-CQ⁴-N(R¹⁰)-,
-N(R¹⁰)-C(R⁸;R⁹)-CQ⁴-N(R¹⁰)-, -C(R⁸)=N-N(R¹⁰)-,
-Q³-CQ⁴-C(R⁸,R⁹)-N(R¹⁰)-,
wobei
- Q³, Q⁴ und Q⁵: gleich oder verschieden sind und jeweils für Sauerstoff oder Schwefel stehen,
- R⁸ und R⁹: gleich oder verschieden sind und einzeln für Wasserstoff, Fluor, Chlor, Methyl oder Ethyl stehen oder zusammen für Ethan-1,2-diyl (Dimethylen) stehen, und
- R¹⁰: für Wasserstoff, Hydroxy, für gegebenenfalls durch Cyano, Fluor, Chlor, Methoxy, Ethoxy, Acetyl, Propionyl, Methoxycarbonyl oder Ethoxy-carbonyl substituiertes Methyl, Ethyl, n- oder i-Propyl, n-, i-,s oder t-Butyl, für jeweils gegebenenfalls durch Fluor, Chlor oder Brom substituiertes Propenyl, Butenyl, Propinyl oder Butinyl, für jeweils gegebenenfalls durch Fluor, Chlor, Brom, Methyl oder Ethyl substituiertes Cyclopropyl, Cyclobutyl, Cyclopentyl, Cyclohexyl, Cyclopropylmethyl, Cyclobutylmethyl, Cyclopentylmethyl oder Cyclohexylmethyl, für jeweils gegebenenfalls durch Fluor und/oder Chlor substituiertes Methoxy, Ethoxy, n- oder i-Propoxy, n-, i- oder s-Butoxy, Propenyloxy oder Butenyloxy, oder jeweils gegebenenfalls durch Cyano, Fluor, Chlor, Methyl, Ethyl, Trifluormethyl, Methoxy, Ethoxy, Difluormethoxy oder Trifluormethoxy substituiertes Benzyl oder Benzyloxy steht,
- R⁴: für Amino, Methyl, Ethyl, n- oder i-Propyl oder Cyclopropyl steht,
- R⁵: für jeweils gegebenenfalls durch Fluor und/oder Chlor substituiertes Methyl oder Ethyl steht,
- R⁶: für Wasserstoff, Methyl oder Ethyl steht, und
- R⁷: für Wasserstoff, Methyl, Ethyl, Acetyl, Propionyl, Methoxycarbonyl, Ethoxycarbonyl, Methylaminocarbonyl, Ethylaminocarbonyl, Dimethylaminocarbonyl, Methylsulfonyl oder Ethylsulfonyl steht.

Die oben aufgeführten allgemeinen oder in Vorzugsbereichen angegebenen Restedefinitionen gelten sowohl für die Endprodukte der Formel (I) als auch entsprechend für die jeweils zu Herstellung benötigten Ausgangsstoffe bzw. Zwischenprodukte. Diese Restedefinitionen können untereinander, also auch zwischen den angegebenen Bereichen bevorzugter Verbindungen, beliebig kombiniert werden.

Man erhält die neuen substituierten Diazacyclohexandi(thi)one der allgemeinen Formel (I), wenn man Uracile der allgemeinen Formel (II) 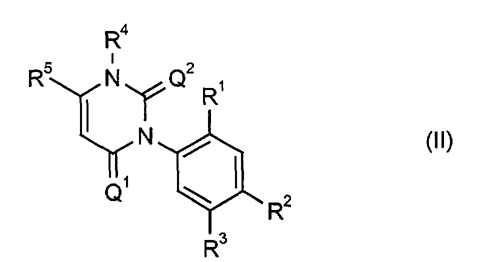 in welcher
- Q¹, Q², R¹, R², R³, R⁴ und R⁵: die oben angegebenen Bedeutungen haben,
mit Ammoniak gegebenenfalls in Gegenwart eines Verdünnungsmittels umsetzt und gegebenenfalls die so erhaltenen Verbindungen der Formel (I), in welcher und R⁷ für Wasserstoff stehen, nach üblichen Methoden mit Alkylierungs-, Acylierungsoder Sulfonylierungsmitteln umsetzt.

Die neuen substituierten Diazacyclohexandi(thi)one der allgemeinen Formel (I) zeichnen sich durch starke und selektive herbizide Wirksamkeit aus.

In den Definitionen sind die gesättigten oder ungesättigten Kohlenwasserstoffketten, wie Alkyl, Alkandiyl, Alkenyl oder Alkinyl - auch in Verbindung mit Heteroatomen, wie in Alkoxy, Alkylthio oder Alkylamino - jeweils geradkettig oder verzweigt.

Halogen steht im allgemeinen für Fluor, Chlor, Brom oder Iod, vorzugsweise für Fluor, Chlor oder Brom, insbesondere für Fluor oder Chlor.

Beispiele für die erfindungsgemäßen Verbindungen der Formel (I) sind in den nachstehenden Gruppen aufgeführt.

### Gruppe 1

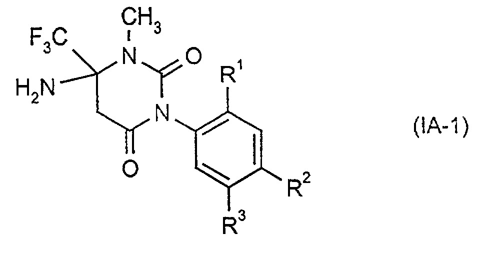

R¹, R² und R³ haben dabei die in der nachstehenden Auflistung angegebenen Bedeutungen

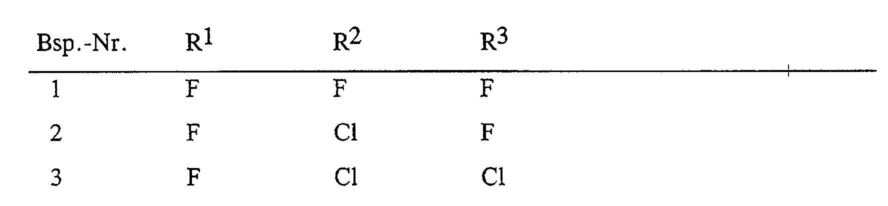

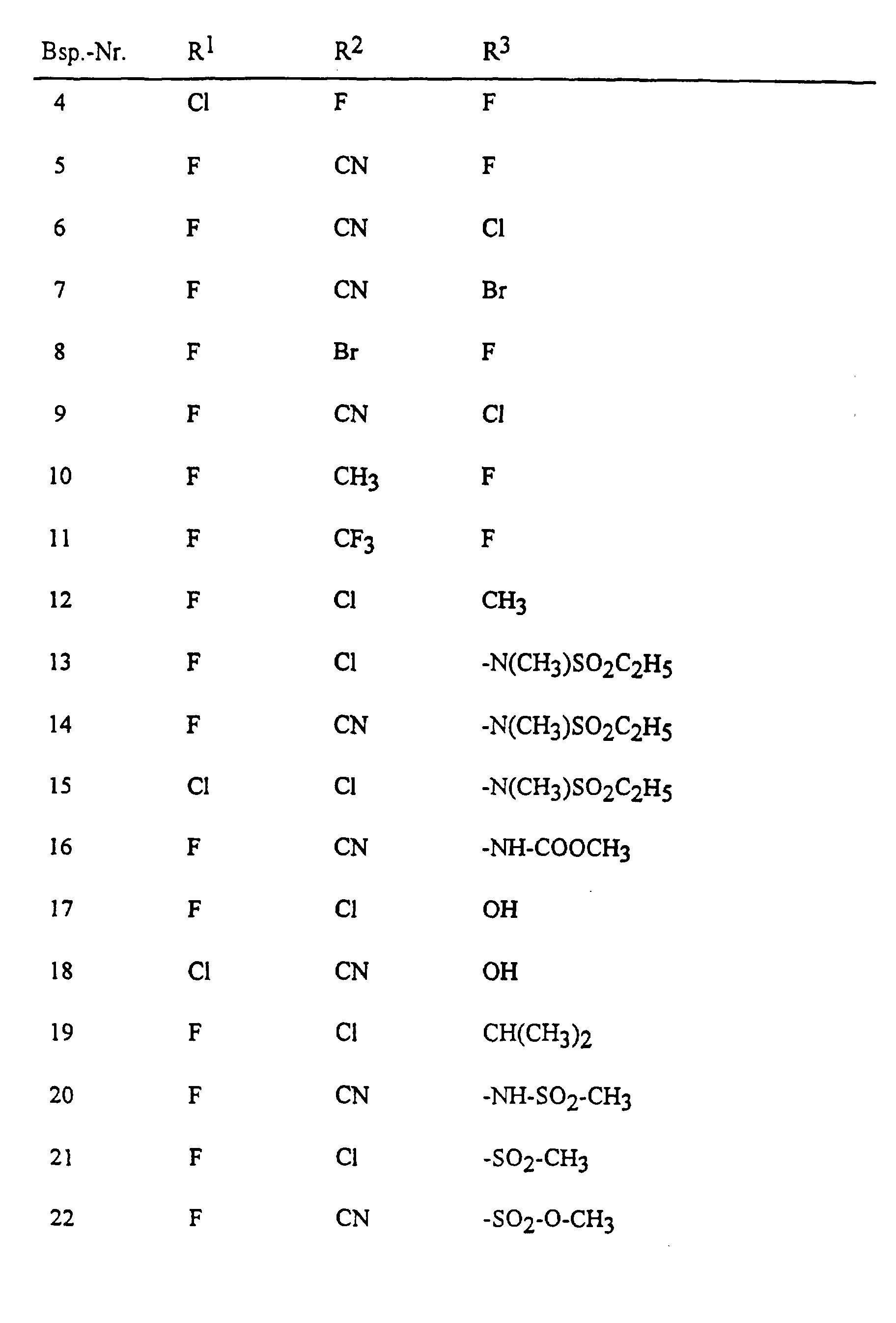

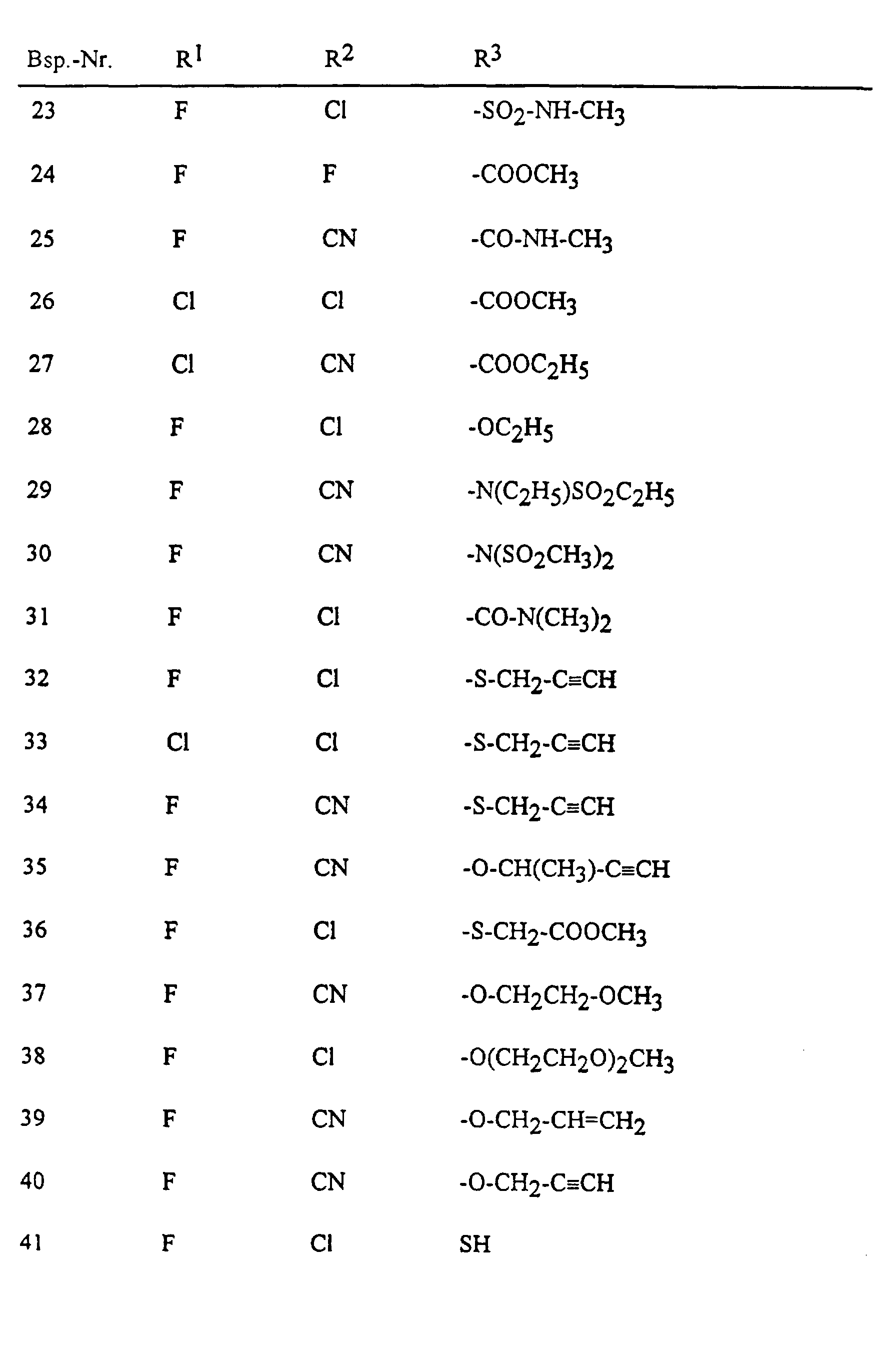

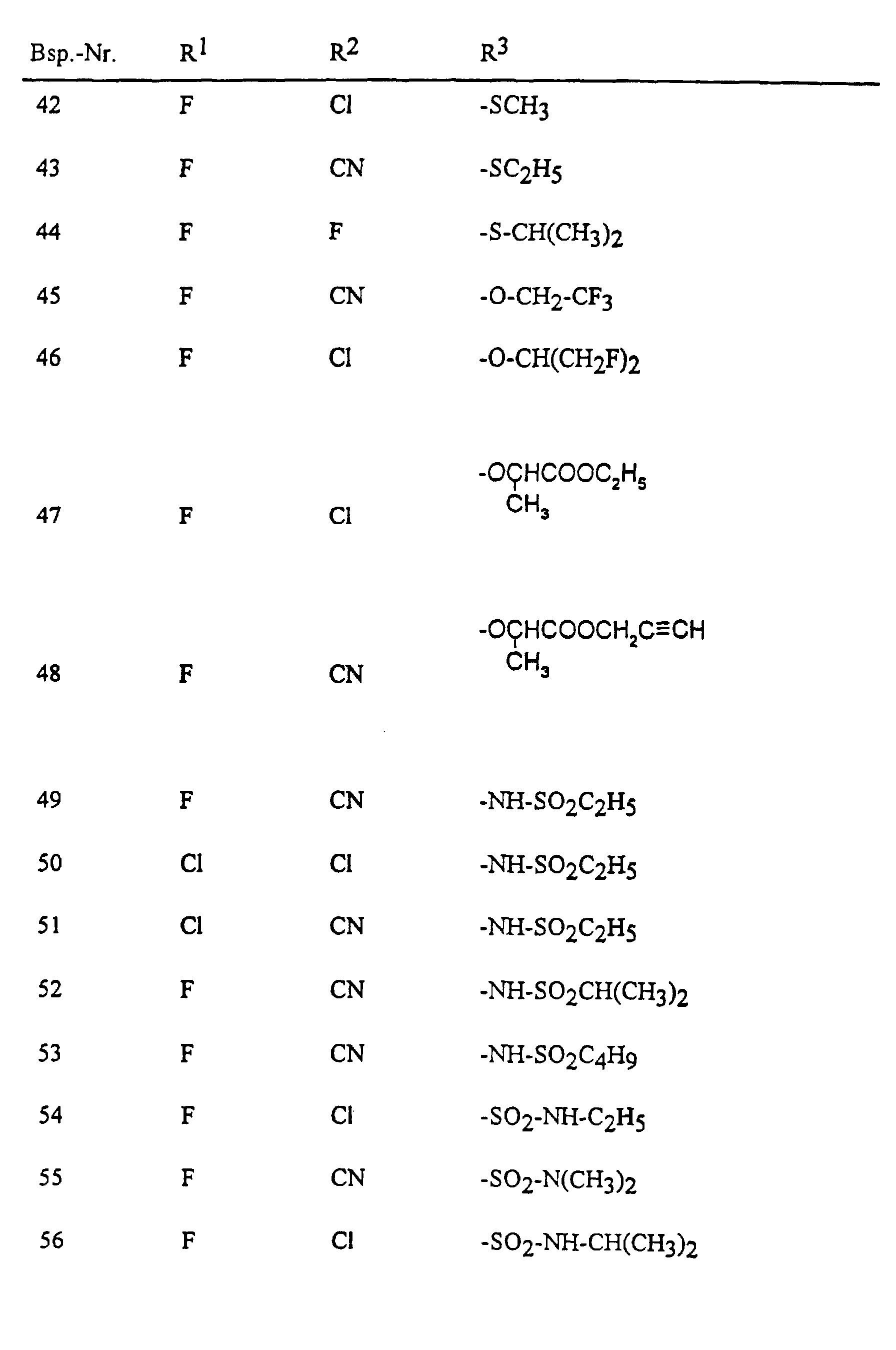

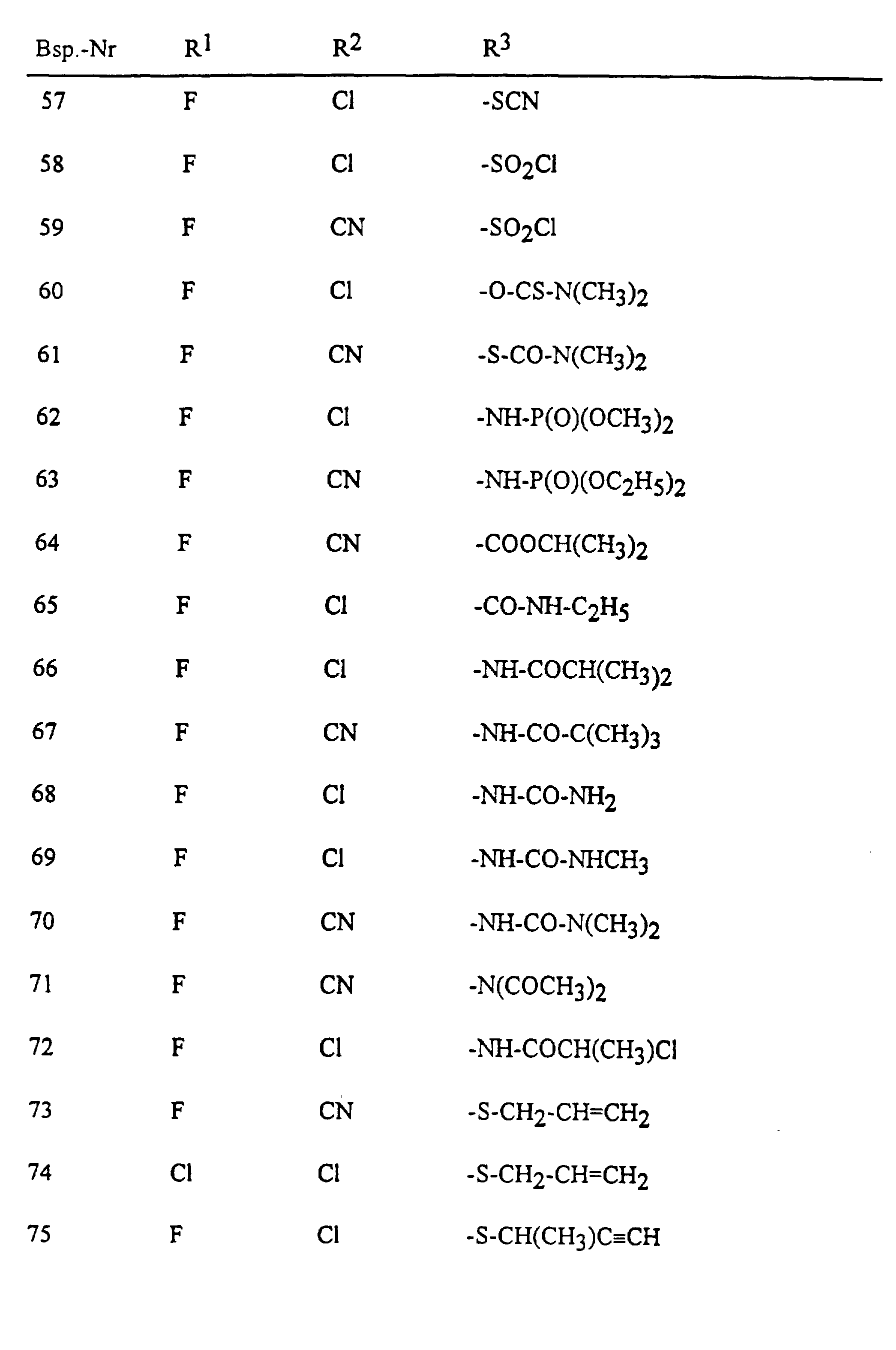

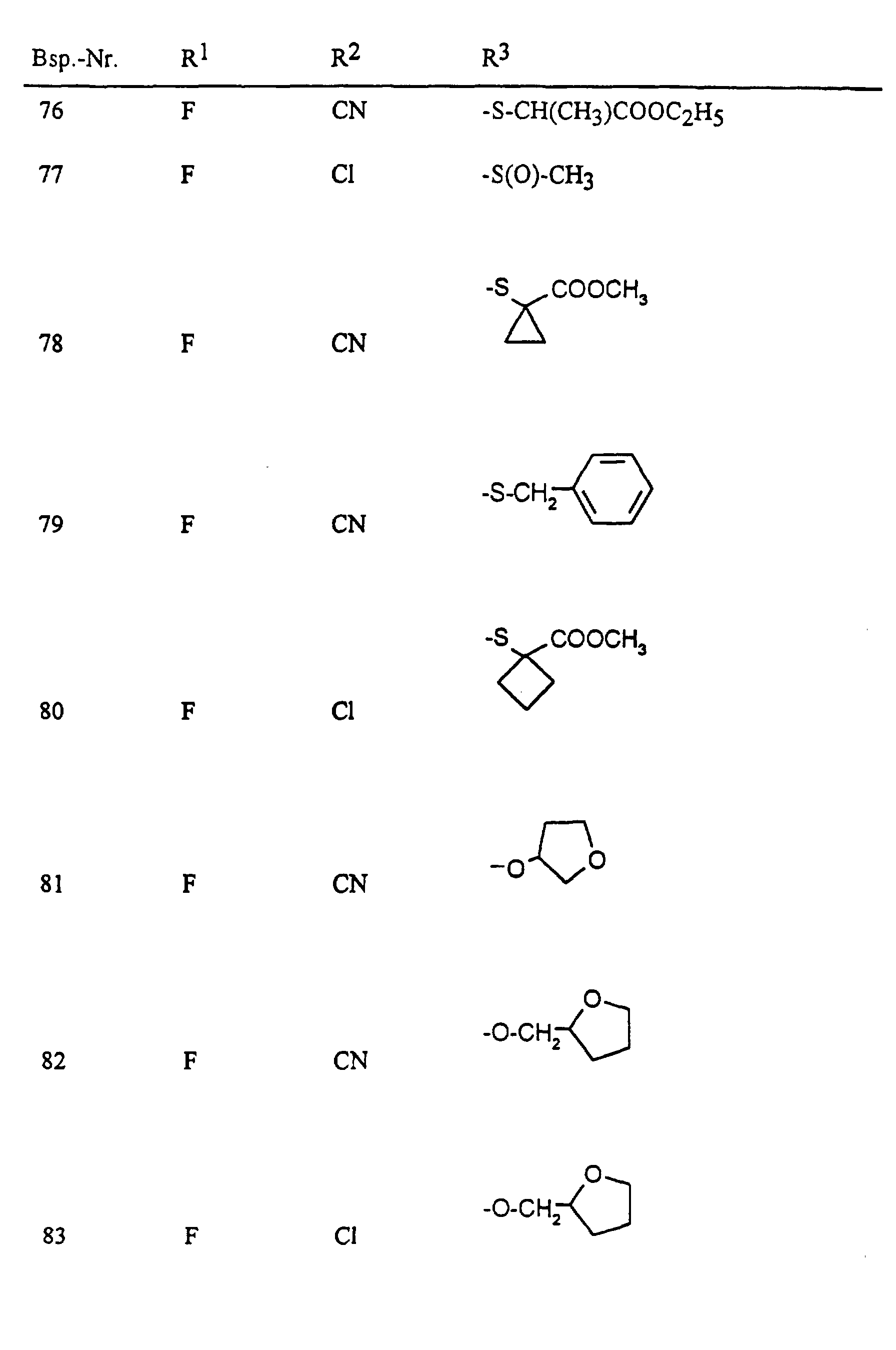

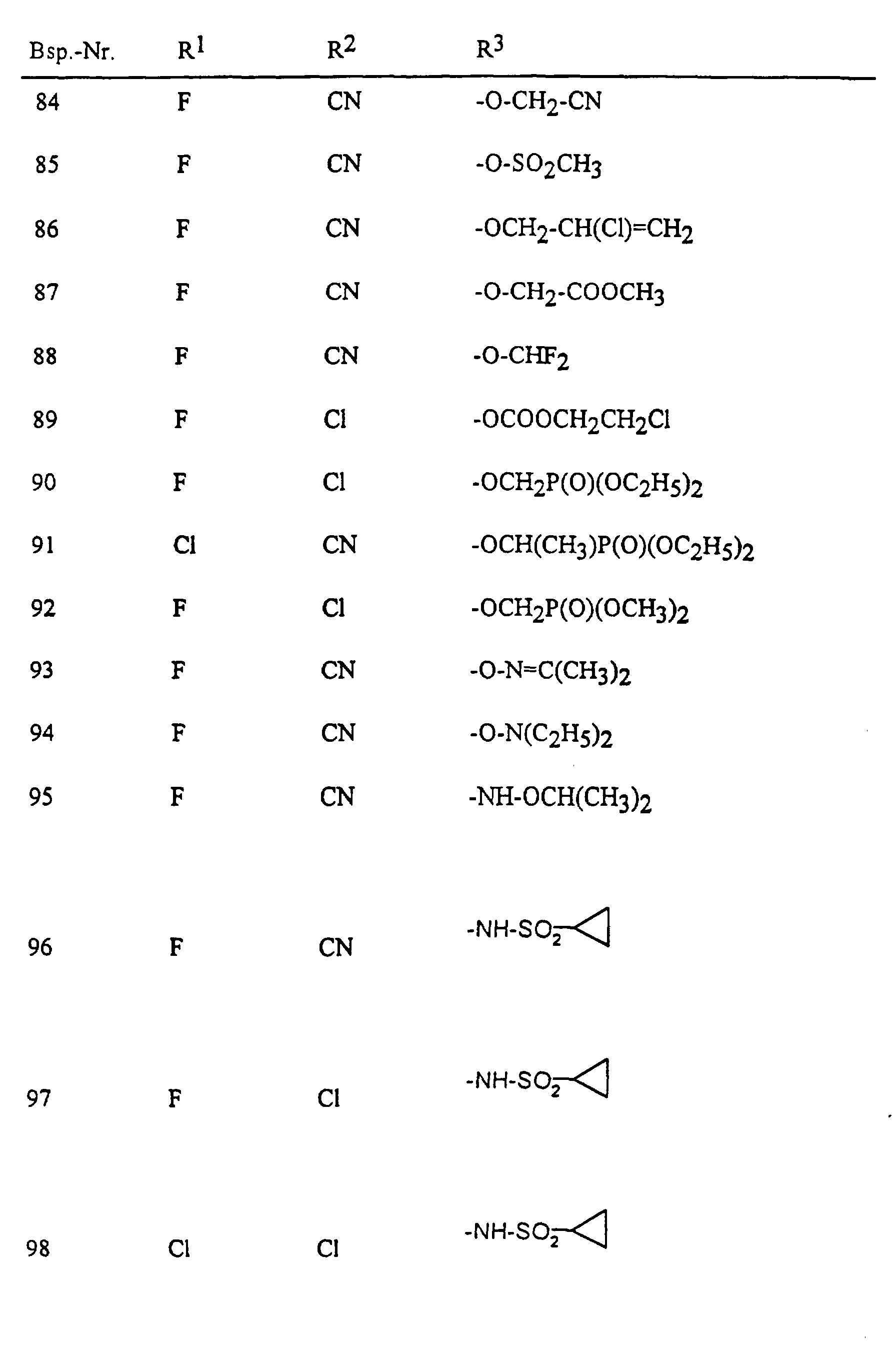

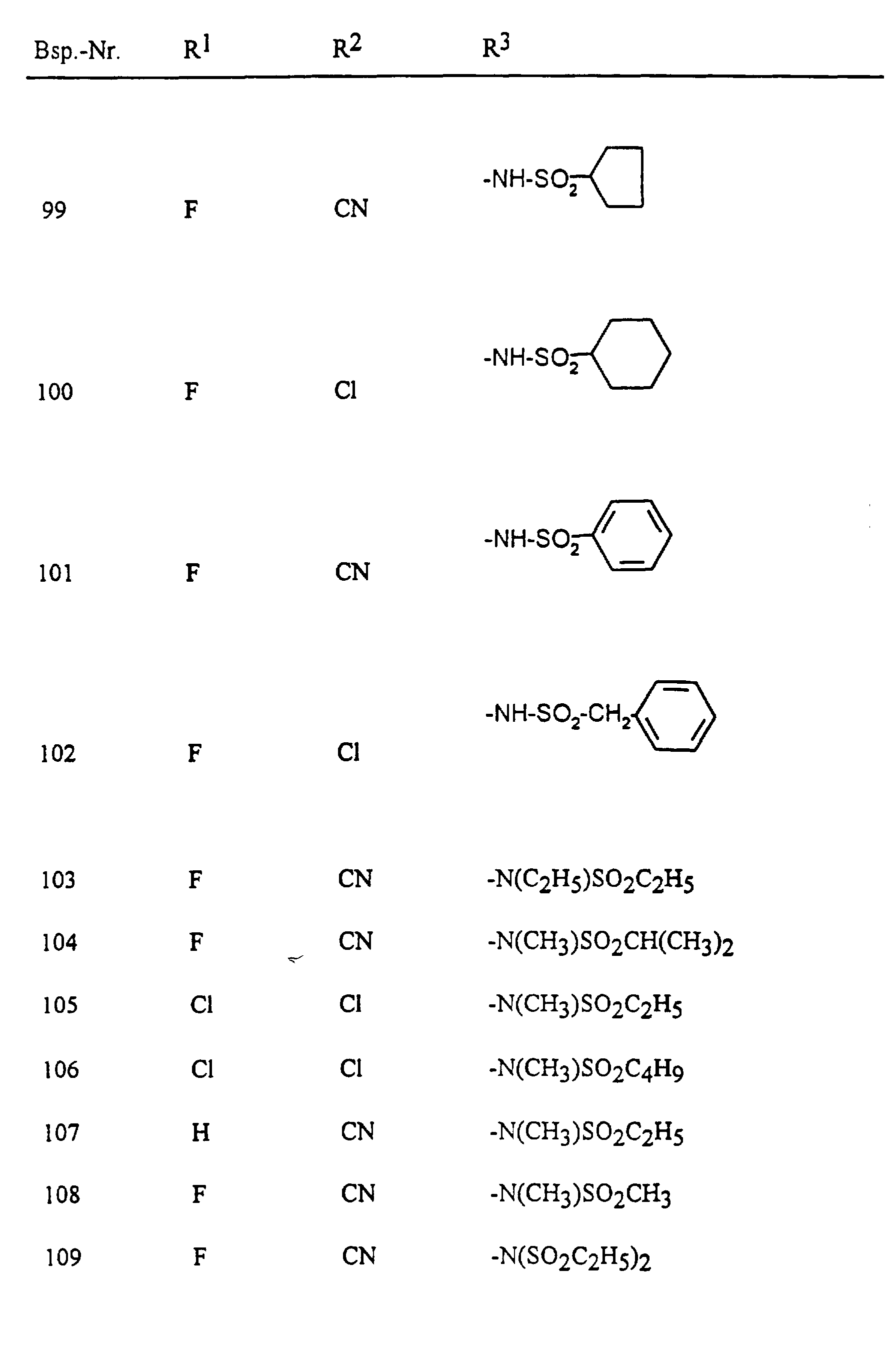

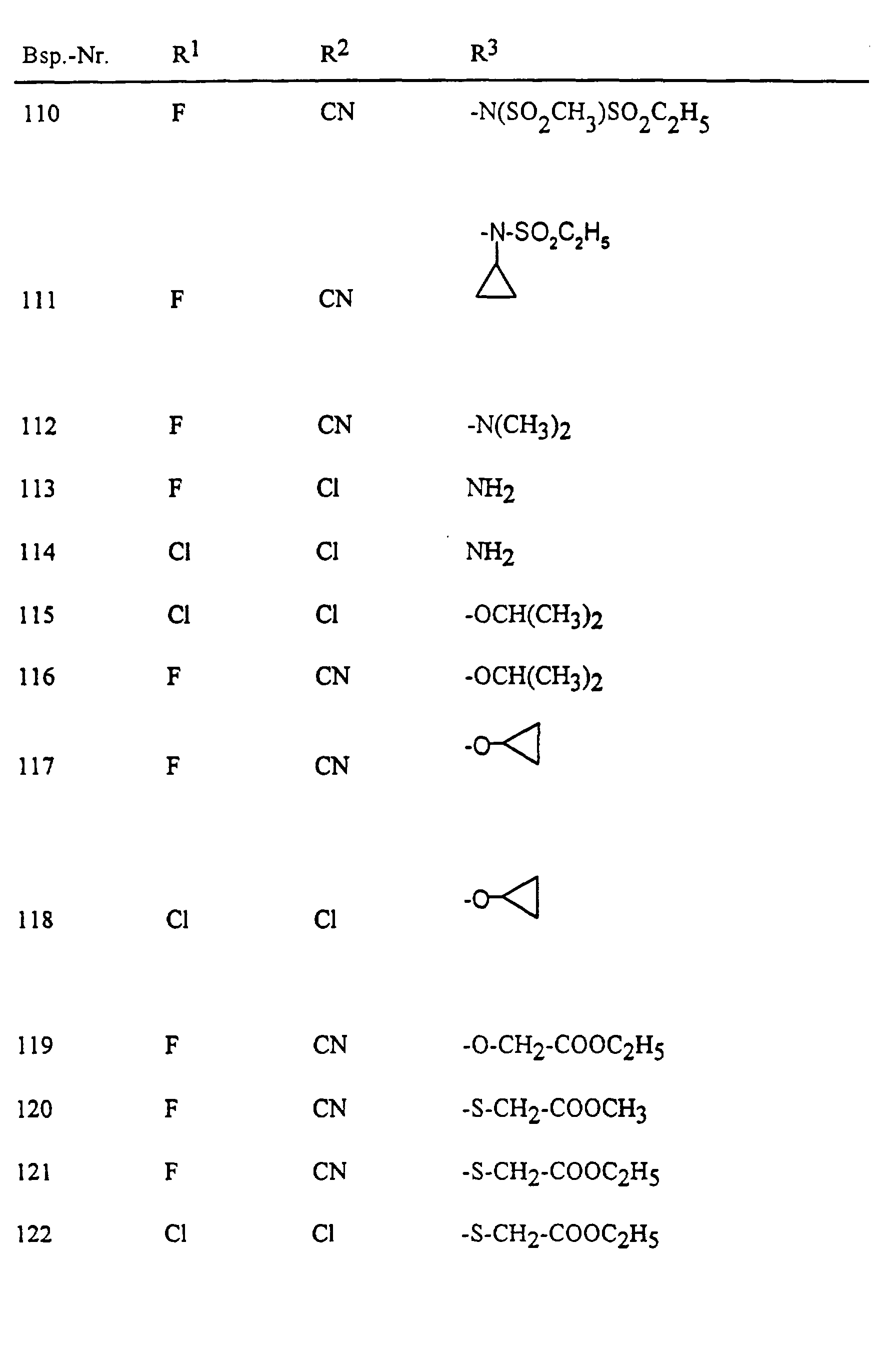

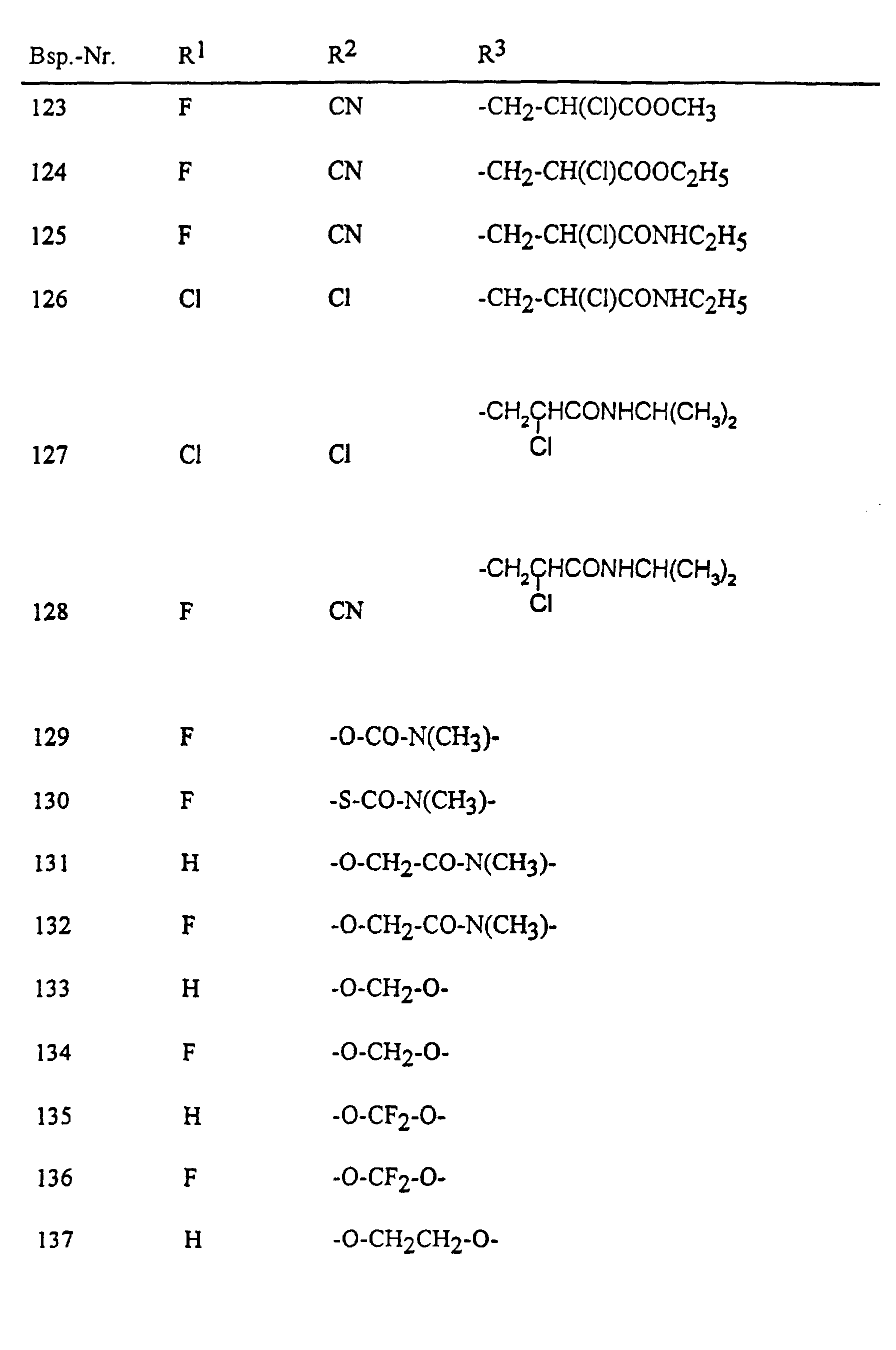

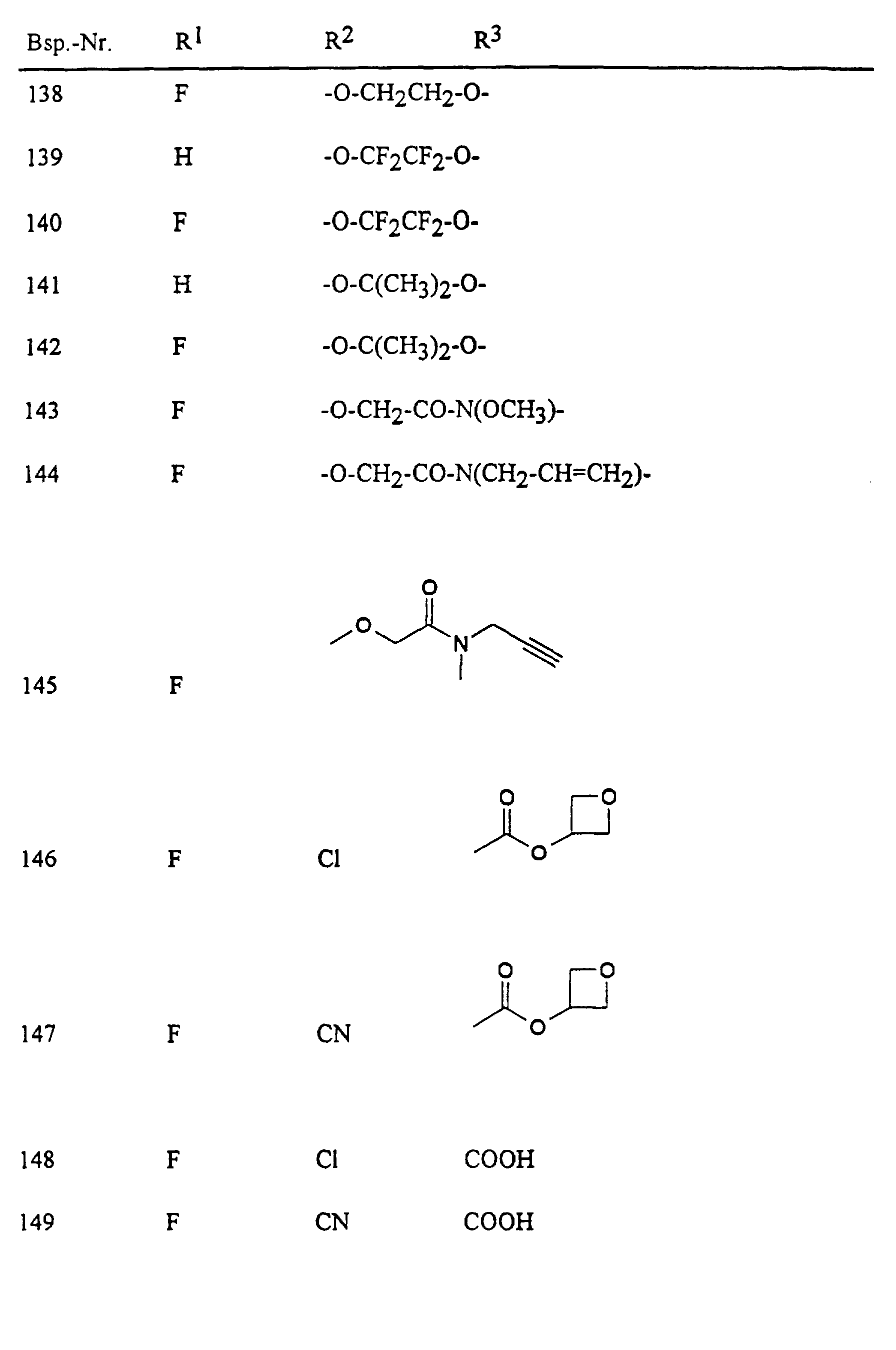

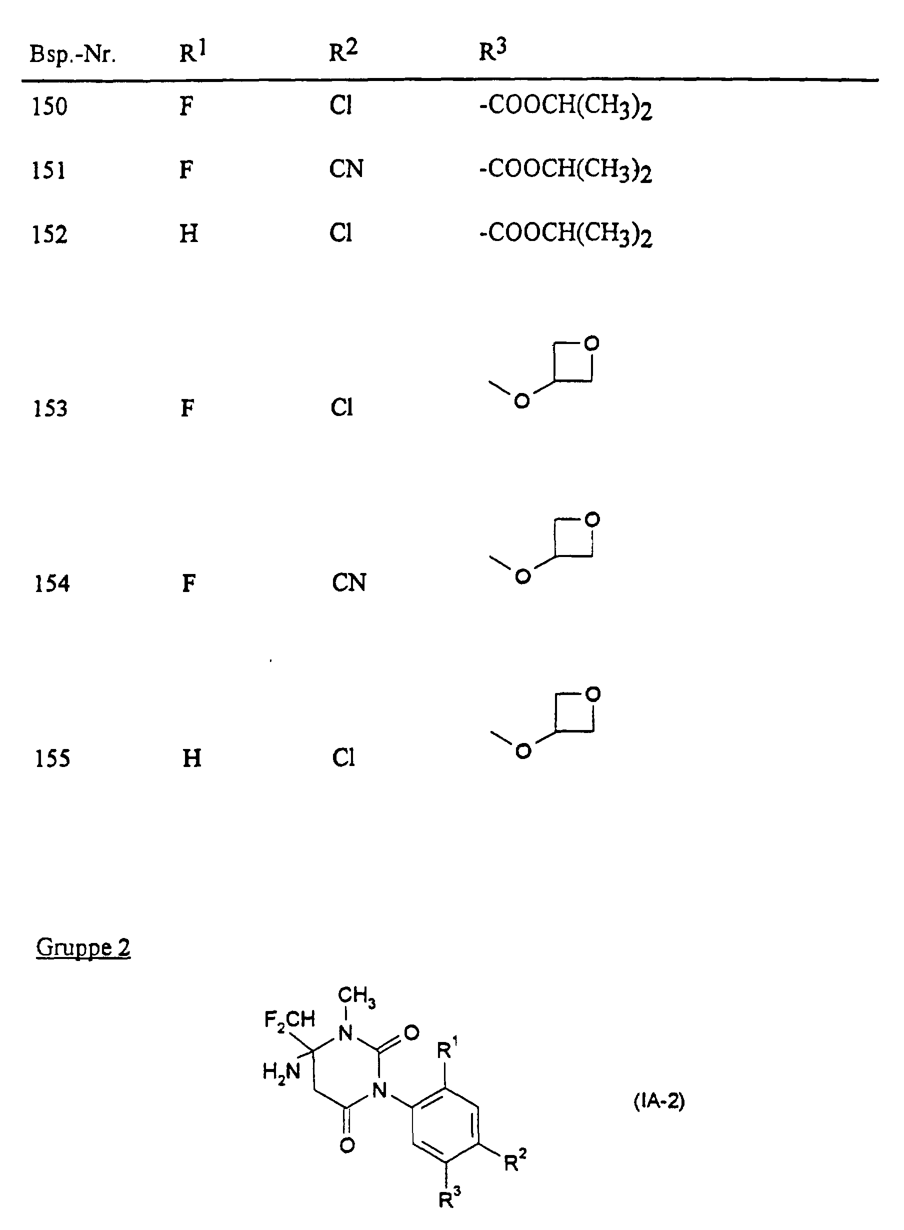

R¹, R² und R³ haben hierbei beispielhaft die oben in Gruppe 1 angegebenen Bedeutungen.

### Gruppe 3

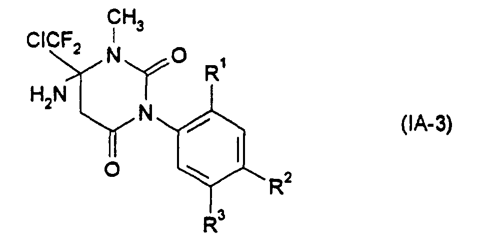

R¹, R² und R³ haben hierbei beispielhaft die oben in Gruppe 1 angegebenen Bedeutungen.

### Gruppe 4

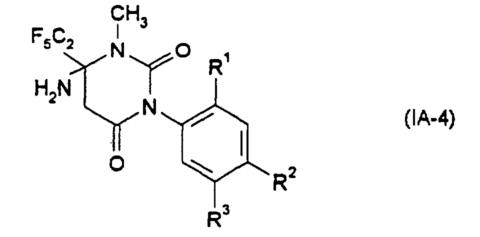

R¹, R² und R³ haben hierbei beispielhaft die oben in Gruppe 1 angegebenen Bedeutungen.

### Gruppe 5

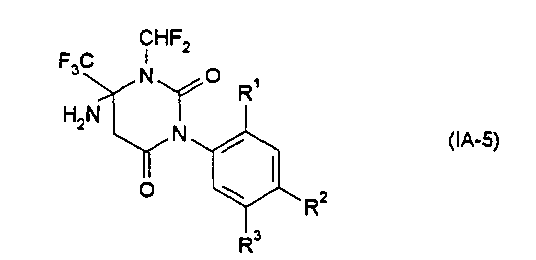

R¹, R² und R³ haben hierbei beispielhaft die oben in Gruppe 1 angegebenen Bedeutungen.

### Gruppe 6

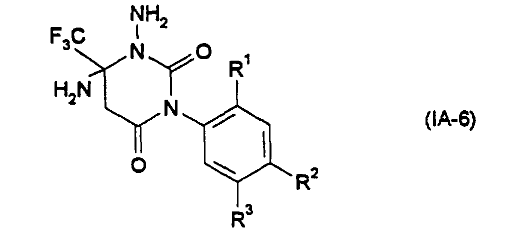

R¹, R² und R³ haben hierbei beispielhaft die oben in Gruppe 1 angegebenen Bedeutungen.

Verwendet man beispielsweise 1-(2,4-Dichlor-5-methoxy-phenyl)-3,6-dihydro-2,6-dioxo-3-methyl-4-trifluormethyl-1(2H)-pyrimidin und Ammoniak als Ausgangsstoffe, so, kann der Reaktionsablauf beim erfindungsgemäßen Verfahren durch das folgende Formelschema skizziert werden:

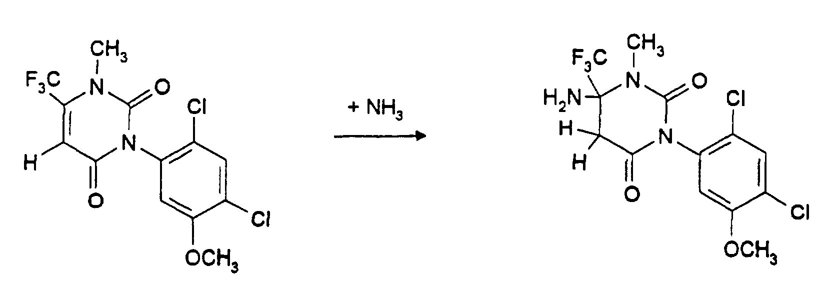

Die beim erfindungsgemäßen Verfahren zur Herstellung der Verbindungen der allgemeinen Formel (I) als Ausgangsstoffe zu verwendenden Uracile sind durch die Formel (II) allgemein definiert. In der Formel (II) haben R¹, R², R³, R⁴ und R⁵ vorzugsweise bzw. insbesondere diejenigen Bedeutungen, die bereits oben im Zusammenhang mit der Beschreibung der Verbindungen der Formel (I) als bevorzugt bzw. als insbesondere bevorzugt für R¹, R², R³, R⁴ und R⁵ angegeben wurden.

Die Ausgangsstoffe der Formel (II) sind bekannt und/oder können nach bekannten Verfahren hergestellt werden (vgl. US-P 5084084, US-P 5127935, US-P 5154755, US-P 5169430, DE-A 4327743 vom 18.8.1993, Herstellungsbeispiele).

Die Ausgangsstoffe der Formel (II) können gemäß folgendem Formelschema hergestellt werden:

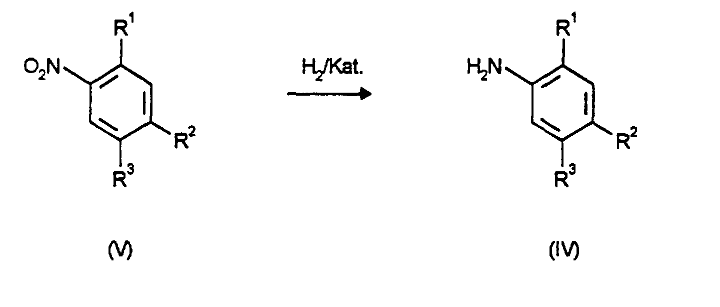

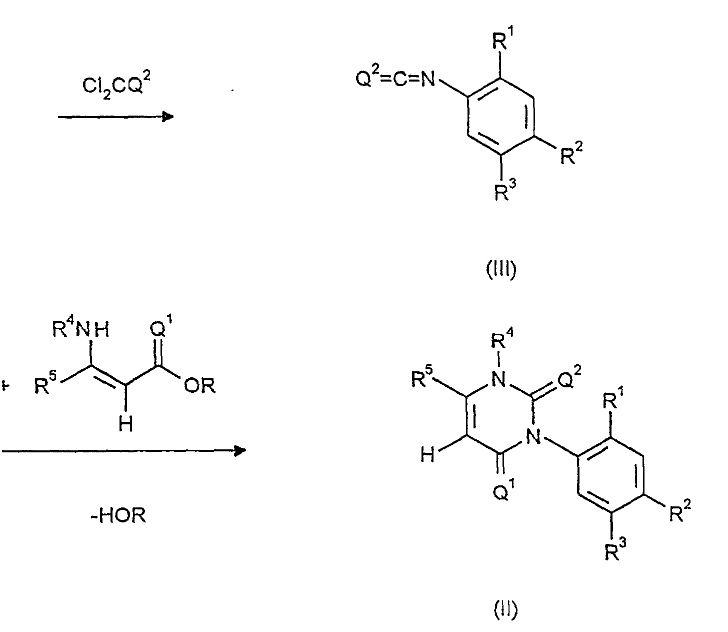

Von den Verbindungen der Formeln (II), (III) und (IV) sind jeweils diejenige, bei denen R¹ für Fluor oder Chlor steht, R² für Cyano oder Methyl steht und R³ für Amino oder Nitro steht noch nicht aus der Literatur bekannt.

An Stelle der Verbindungen der Formel (III) können hierbei mit dem gleichen Ergebnis auch Verbindungen der Formel (VI) 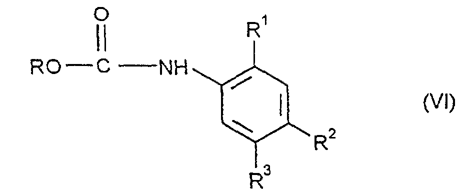 wobei R¹, R² und R³ wie vorausgehend definiert sind und R für Alkyl (insbesondere Methyl oder Ethyl), Aryl (insbesondere Phenyl) oder Aralkyl (insbesondere Benzyl) steht, eingesetzt werden.

Als Verdünnungsmittel zur Durchführung des erfindungsgemäßen Verfahrens kommen die üblichen organischen Lösungsmittel in Betracht. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Dichlorbenzol, Petrolether, Hexan, Cyclohexan, Dichlormethan, Chloroform, Tetrachlormethan; Ether, wie Diethylether, Diisopropylether, Dioxan, Tetrahydrofuran oder Ethylenglykoldimethyl- oder -diethylether; Ketone, wie Aceton, Butanon oder Methyl-isobutyl-keton; Nitrile, wie Acetonitril, Propionitril oder Benzonitril; Amide, wie N,N-Dimethylformamid, N,N-Dimethylacetamid, N-Methylformanilid, N-Methylpyrrolidon oder Hexamethylphosphorsäuretriamid; Ester wie Essigsäuremethylester oder Essigsäureethylester, Sulfoxide, wie Dimethylsulfoxid, Alkohole, wie Methanol, Ethanol, n- oder i-Propanol, Ethylenglykolmonomethylether, Ethylenglykolmonoethylether, Diethylenglykolmonomethylether, Diethylenglykolmonoethylether, deren Gemische mit Wasser oder reines Wasser.

Die Reaktionstemperaturen können bei der Durchführung des erfindungsgemäßen Verfahrens in einem größeren Bereich variiert werden. Im allgemeinen arbeitet man bei Temperaturen zwischen -100°C und +40°C, vorzugsweise bei Temperaturen zwischen -80°C und +20°C, insbesondere bei Temperaturen zwischen -60°C und 0C. Das erfindungsgemäße Verfahren wird im allgemeinen unter Normaldruck durchgeführt. Es ist jedoch auch möglich, unter erhöhtem oder vermindertem Druck - im allgemeinen zwischen 0,1 bar und 10 bar - zu arbeiten.

Zur Durchführung des erfindungsgemäßen Verfahrens werden die jeweils benötigten Ausgangsstoffe im allgemeinen in angenähert äquimolaren Mengen eingesetzt. Es ist jedoch auch möglich, eine der beiden jeweils eingesetzten Komponenten in einem größeren Uberschuß zu verwenden. Die Reaktionen werden im allgemeinen in einem geeigneten Verdünnungsmittel durchgeführt, und das Reaktionsgemisch wird mehrere Stunden bei der jeweils erforderlichen Temperatur gerührt. Die Aufarbeitung erfolgt bei dem erfindungsgemäßen Verfahren jeweils nach üblichen Methoden (vgl. die Herstellungsbeispiele).

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewandten Menge ab.

Die erfindungsgemäßen Wirkstoffe können z.B. bei den folgenden Pflanzen verwendet werden:
Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea, Trifolium, Ranunculus, Taraxacum.
Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.
Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.
Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen, auf Zier- und Sportrasen und Weideflächen und zur selektiven Unkrautbekämpfung in einjährigen Kulturen eingesetzt werden.

Die erfindungsgemäßen Verbindungen der Formel (I) eignen sich insbesondere zur selektiven Bekämpfung von monokotylen und dikotylen Unkräutern in monokotylen und dikotylen Kulturen sowohl im Vorauflauf- als auch im Nachauflauf-Verfahren.

Die Wirkstoffe können in die üblichen Formulierungen übergeführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:

z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide infrage, beispielsweise Anilide, wie z.B. Diflufenican und Propanil; Arylcarbonsäuren, wie z.B. Dichlorpicolinsäure, Dicamba und Picloram; Aryloxyalkansäuren, wie z.B. 2,4 D, 2,4 DB, 2,4 DP, Fluroxypyr, MCPA, MCPP und Triclopyr; Aryloxy-phenoxy-alkansäureester, wie z.B. Diclofop(-methyl), Fenoxaprop(-ethyl), Fluazifop(-butyl), Haloxyfop(-methyl) und Quizalofop(-ethyl); Azinone, wie z.B. Chloridazon und Norflurazon; Carbamate, wie z B. Chlorpropham, Desmedipham, Phenmedipham und Propham; Chloracetanilide, wie z.B. Alachlor, Acetochlor, Butachlor, Metazachlor, Metolachlor, Pretilachlor und Propachlor; Dinitroaniline, wie z.B. Oryzalin, Pendimethalin und Trifluralin; Diphenylether, wie z.B. Acifluorfen, Bifenox, Chlormethoxynil (X-52), Chlornitrofen, Fluoroglycofen, Fomesafen, Halosafen, Lactofen, Nitrofen und Oxyfluorfen; Harnstoffe, wie z.B. Chlortoluron, Cumyluron (JC-940), Diuron, Dymron (Daimuron), Fluometuron, Isoproturon, Linuron und Methabenzthiazuron; Hydroxylamine, wie z.B. Alloxydim, Clethodim, Cycloxydim, Sethoxydim und Tralkoxydim; Imidazolinone, wie z.B. Imazethapyr, Imazamethabenz, Imazapyr und Imazaquin; Nitrile, wie z.B. Bromoxynil, Dichlobenil und Ioxynil; Oxyacetamide, wie z.B. Mefenacet; Sulfonylharnstoffe, wie z.B. AC-014 (AC-322140), Amidosulfuron, Bensulfuron(-methyl), Chlorimuron(-ethyl), Chlorsulfuron, Cinosulfuron, DPX-47, HOE-404, Imazosulfuron, Metsulfuron(-methyl), Nicosulfuron, Primisulfuron, Pyrazosulfuron(-ethyl), Thifensulfuron(-methyl), Triasulfuron und Tribenuron(-methyl); Thiolcarbamate, wie z.B. Butylate, Cycloate, Diallate, Dimepiperate, EPTC, Esprocarb, Molinate, Prosulfocarb, Thiobencarb (Benthiocarb) und Triallate; Triazine, wie z.B. Atrazin, Cyanazin, Dimethametryn, Prometryne, Simazin, Simetryne, Terbutryne und Terbutylazin; Triazinone, wie z.B. Hexazinon, Metamitron und Metribuzin; Sonstige, wie z.B. Aminotriazol, Benfuresate, Bensulide, Bentazone, Benzofenap, Bromobutide, Butamifos, Cafenstrole (CH-900), Cinmethylin, Clomazone, Clomeprop, Clopyralid, DEH-112, Difenzoquat, Dimethenamid, Dithiopyr, Ethofumesate, Flumetsulam, Fluorochloridone, Glufosinate, Glyphosate, Amiprophos(-methyl), Anilofos, Etobenzanid (HW-52), Isoxaben, KPP-314, KUH-833, KUH-911, KUH-920, MK-243, Naproanilide, NSK-850, Oxadiazon, Piperophos, Propanil, Pyrazolate, Pyrazoxyfen, Pyributicarb, Pyridate, Quinchlorac, Quinmerac, Sulphosate und Tridiphane.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden. Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 10 g und 10 kg Wirkstoff pro Hektar Bodenfläche, vorzugsweise zwischen 50 g und 5 kg pro ha.

Die Herstellung und die Verwendung der erfindungsgemäßen Wirkstoffe geht aus den nachfolgenden Beispielen hervor.

### Herstellungsbeispiele:

### Beispiel 1

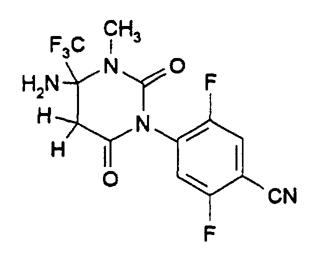

In eine Mischung aus 0,80 g (2,4 mMol) 1-(4-Cyano-2,5-difluor-phenyl)-3,6-dihydro-2,6-dioxo-3-methyl-4-trifluormethyl-1(2H)-pyrimidin und 20 ml Tetrahydrofuran wird bei -60°C unter Rühren 10 Minuten lang Ammoniak (Gas) eingeleitet. Man lässt die Mischung unter Rühren über Nacht (ca. 15 Stunden) auf Raumtemperatur (ca. 20°C) kommen, verdünnt dann mit Diethylether auf etwa das doppelte Volumen, wäscht die Lösung mit 5%iger wässriger Natriumdihydrogenphosphat-Lösung, trocknet über Natriumsulfat und filtriert. Vom Filtrat wird dann das Lösungsmittel im Wasserstrahlvakuum sorgfältig abdestilliert.

Man erhält 0,70 g (93% der Theorie) 1-(4-Cyano-2,5-difluor-phenyl)-4-amino-2,6-dioxo-3-methyl-4-trifluormethyl-1,3-diaza-cyclohexan als kristallinen Rückstand vom Schmelzpunkt 115°C.

Analog Beispiel 1 sowie entsprechend der allgemeinen Beschreibung des erfindungsgemäßen Herstellungsverfahrens können beispielsweise auch die in der nachstehenden Tabelle 1 aufgeführten Verbindungen der Formel (I) hergestellt werden.

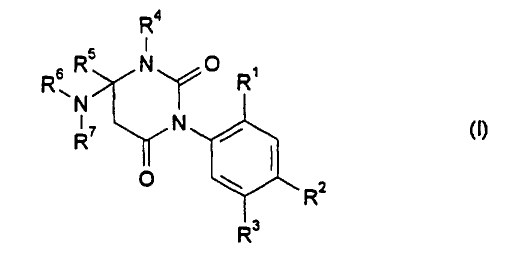

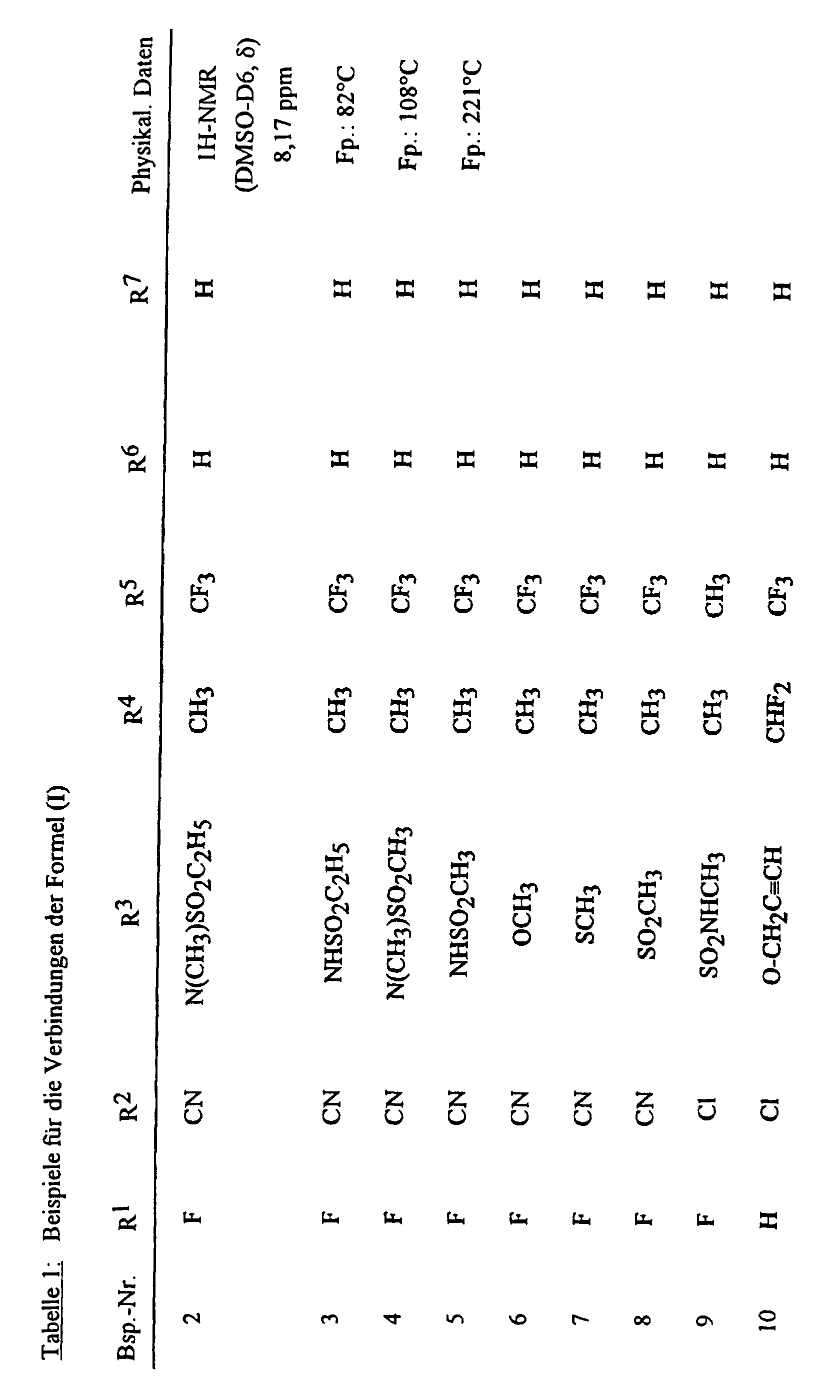

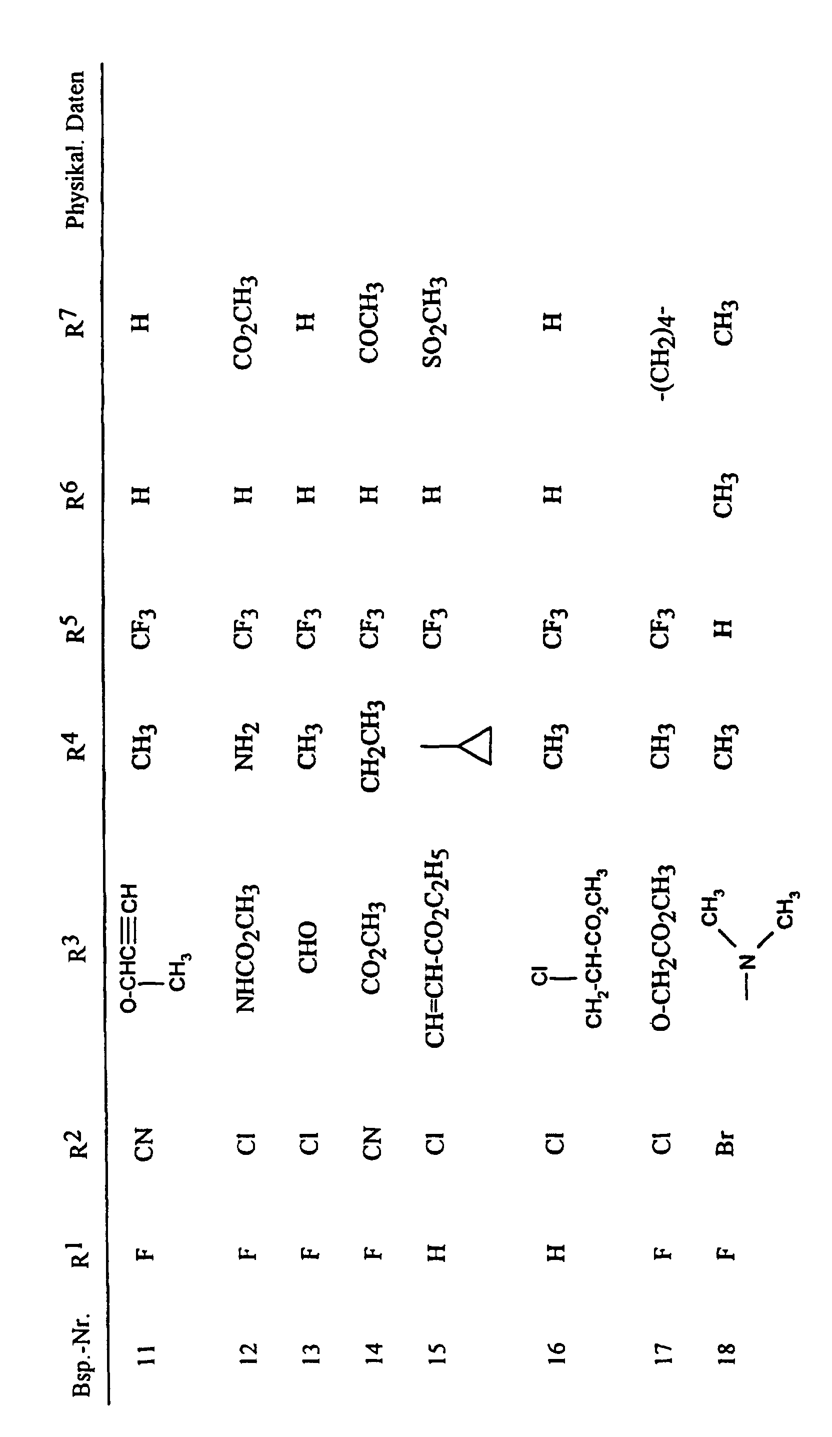

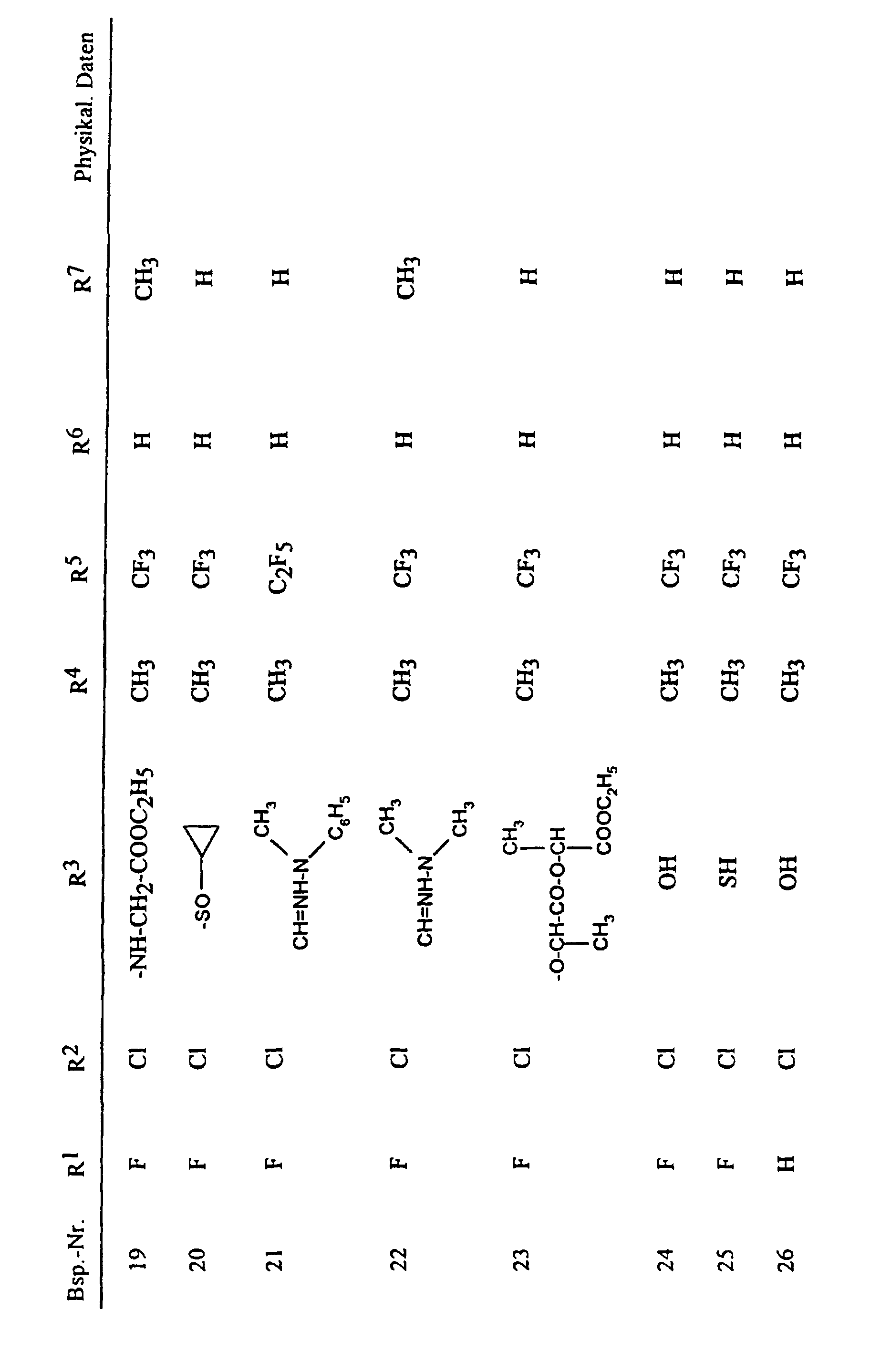

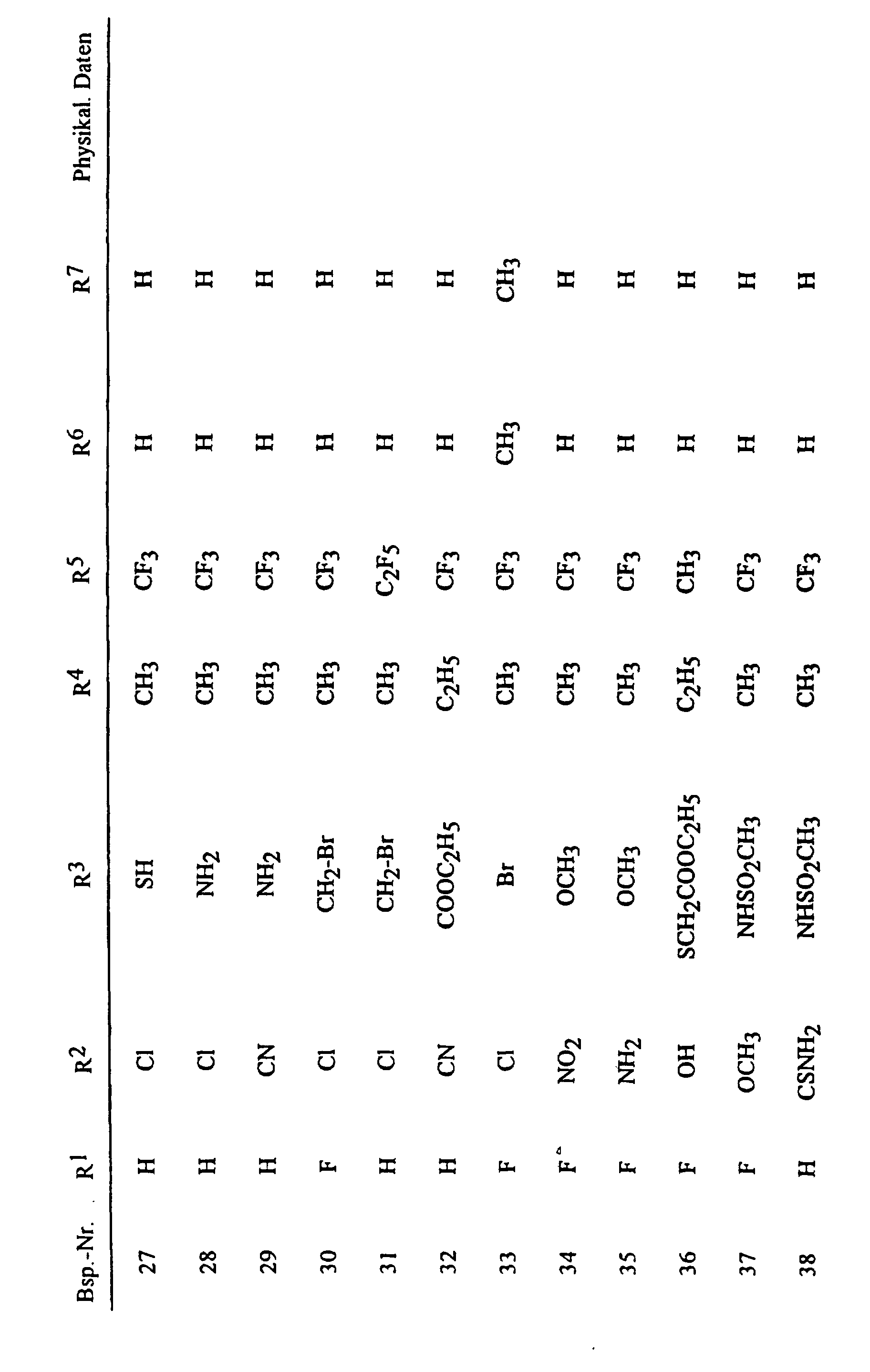

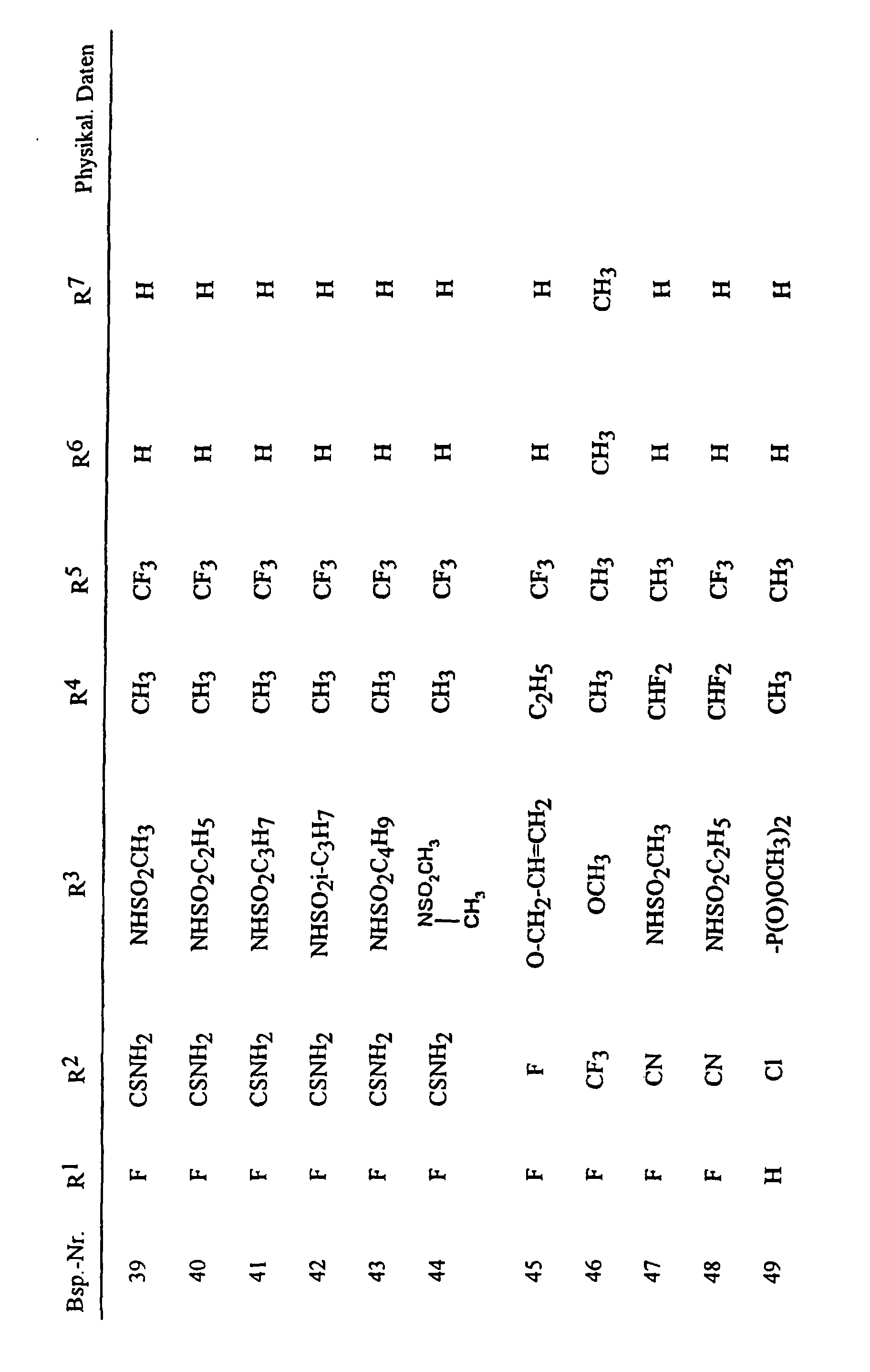

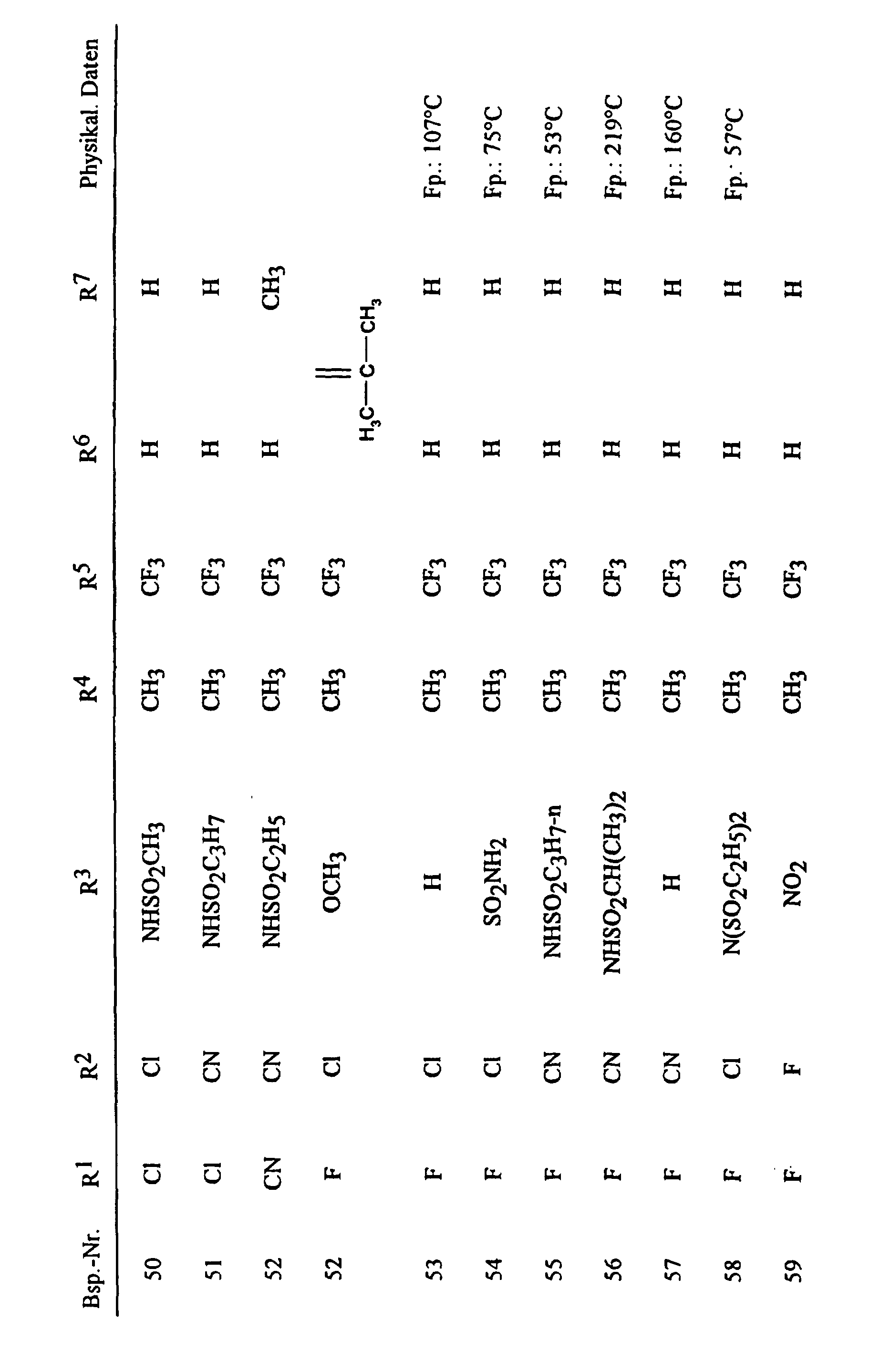

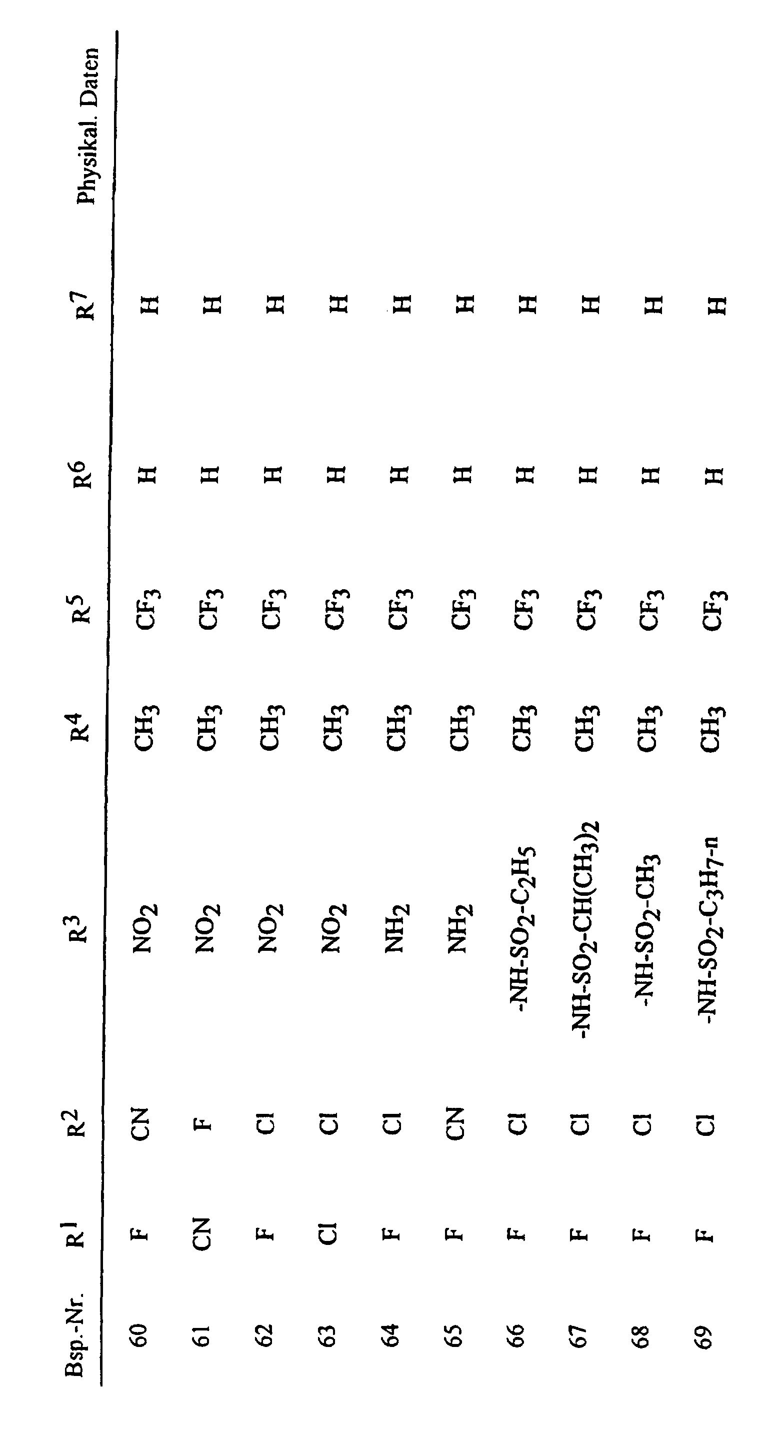

### Ausgangsstoffe der Formel (II):

### Beispiel (II-1)

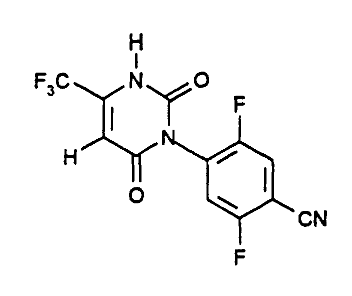

1,8 g (10 mMol) 3-Amino-4,4,4-trifluor-crotonsäureethylester werden in 30 ml Dimethylformamid und 2 ml Toluol vorgelegt und bei 0°C bis 5°C mit 0,3 g (10 mMol) Natriumhydrid (80%ig) versetzt. Das Gemisch wird 30 Minuten bei 0°C bis 5°C gerührt. Nach Abkühlen der Mischung auf -70°C werden 0,9 g (5 mMol) 4-Cyano-2,5-difluor-phenylisocyanat - gelöst in 10 ml Toluol - dazugegeben und das Gemisch wird 150 Minuten bei -60°C bis -70°C gerührt. Nach Entfemen des Kühlbades werden 2 ml Essigsäure dazugegeben. Dann wird mit Wasser auf etwa das doppelte Volumen verdünnt und mit Essigsäureethylester extrahiert. Die organische Phase wird eingeengt und der Rückstand mit Diisopropylether zur Kristallisation gebracht.

Man erhält 1,1 g (69% der Theorie) 1-(4-Cyano-2,5-difluor-phenyl)-3,6-dihydro-2,6-dioxo-4-trifluormethyl-1(2H)-pyrimidin vom Schmelzpunkt 194°C.

### Beispiel (II-2)

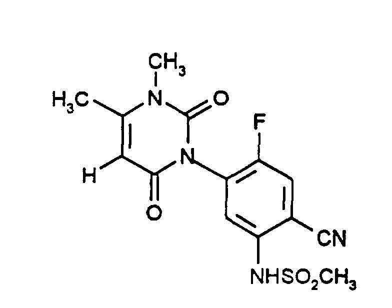

Eine Mischung aus 0,83 g (3 mMol) 1-(4-Cyano-2,5-difluor-phenyl)-3,6-dihydro-2,6-dioxo-3,4-dimethyl-1(2H)-pyrimidin, 0,32 g (3 mMol) Methansulfonamid, 0,6 g Kaliumcarbonat und 10 ml Dimethylsulfoxid wird 10 Stunden auf 120°C erhitzt. Nach Abkühlen wird die Mischung auf Eiswasser gegossen und mit 2N-Salzsäure angesäuert. Dann wird mit Essigsäureethylester extrahiert, die organische Phase mit Wasser gewaschen, mit Natriumsulfat getrocknet und filtriert. Vom Filtrat wird das Lösungsmittel im Wasserstrahlvakuum sorgfältig abdestilliert.

Man erhält 0,8 g (76% der Theorie) 1-(4-Cyano-2-fluor-5-methylsulfonylamino)-3,6-dihydro-2,6-dioxo-3,4-dimethyl-1(2H)-pyrimidin als kristallinen Rückstand (Schmelzpunkt >250°C).

### Anwendungsbeispiele:

### Beispiel A

| Post-emergence-Test | |
|---|---|
| Lösungsmittel | 5 Gewichtsteile Aceton |
| Emulgator | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben so, daß die jeweils gewünschten Wirkstoffmengen pro Flächeneinheit ausgebracht werden. Die Konzentration der Spritzbrühe wird so gewählt, daß in 20001 Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle.

Es bedeuten:
0 % = keine Wirkung (wie unbehandelte Kontrolle)
100 % = totale Vernichtung

In diesem Test zeigt beispielsweise Herstellungsbeispiel (1) bei einer Aufwandmenge von nur 60 g/ha und bei sehr guter Verträglichkeit gegenüber Kulturpflanzen, wie z.B. Gerste (10%), sehr starke Wirkung gegen Unkräuter wie Panicum (90%), Abutilon (100%), Amaranthus (100%), Ambrosia (100%), Chenopodium (100%), Datura (100%) und Solanum (100%).

### Beispiel B

| Post-emergence-Test | |
|---|---|
| Lösungsmittel | 5 Gewichtsteile Aceton |
| Emulgator | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:
0 % = keine Wirkung (wie unbehandelte Kontrolle)
100 % = totale Vernichtung

In diesem Test zeigt Herstellungsbeispiel (1) bei einer Aufwandmenge von nur 30 g/ha und bei sehr guter Verträglichkeit gegenüber Kulturpflanzen, wie z.B. Mais (0%), sehr starke Wirkung gegen Unkräuter wie Digitaria (95%), Abutilon (100%), Chenopodium (100%), Datura (100%), Galinsoga (100%), Matricaria (100%) und Solanum(100%).

## Patentansprüche

1. Diazacyclohexandi(thi)one der allgemeinen Formel (I) 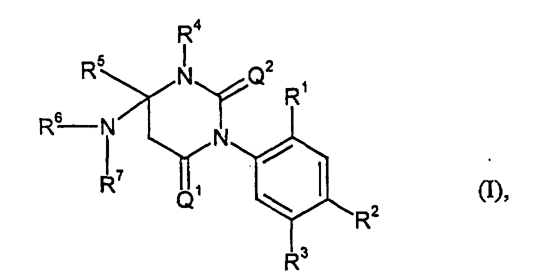 **dadurch gekennzeichnet, daß**
Q¹ für Sauerstoff oder Schwefel steht,
Q² für Sauerstoff oder Schwefel steht,
R¹ für Wasserstoff, Cyano, Nitro, Fluor, Chlor, Brom oder für gegebenenfalls durch Fluor und/oder Chlor substituiertes Alkyl oder Alkoxy mit jeweils 1 bis 4 Kohlenstoffatomen steht,
R² für Wasserstoff, Cyano, Nitro, Thiocarbamoyl, Fluor, Chlor, Brom oder für jeweils gegebenenfalls durch Fluor und/oder Chlor substituiertes Alkyl oder Alkoxy mit jeweils 1 bis 4 Kohlenstoffatomen steht,
R³ für die nachstehende Gruppierung steht,
-A¹-A²-A³
in welcher
A¹ für eine Einfachbindung, für Sauerstoff, Schwefel, -SO-, -SO₂-, -CO- oder die Gruppierung -N-A⁴- steht, worin A⁴ für Wasserstoff, Hydroxy, C₁-C₄-Alkyl, C₁-C₄-Alkoxy, Phenyl, C₁-C₄-Alkylsulfonyl oder Phenylsulfonyl steht,
A¹ weiterhin für jeweils gegebenenfalls durch Fluor, Chlor oder Brom substituiertes C₁-C₆-Alkandiyl, C₂-C₆-Alkendiyl, C₂-C₆-Azaalkendiyl, C₂-C₆-Alkindiyl, C₃-C₆-Cycloalkandiyl, C₃-C₆-Cycloalkendiyl oder Phenylen steht,
A² für eine Einfachbindung, für Sauerstoff, Schwefel, -SO-, -SO₂-, -CO- oder die Gruppierung -N-A⁴- steht, worin A⁴ für Wasserstoff, Hydroxy, C₁-C₄-Alkyl, C₁-C₄-Alkoxy, Phenyl, C₁-C₄-Alkylsulfonyl oder Phenylsulfonyl steht,
A² weiterhin für jeweils gegebenenfalls durch Fluor, Chlor oder Brom substituiertes C₁-C₆-Alkandiyl, C₂-C₆-Alkendiyl, C₂-C₆-Azaalkendiyl, C₂-C₆-Alkindiyl, C₃-C₆-Cycloalkandiyl, C₃-C₆-Cycloalkendiyl oder Phenylen steht,
A³ für Wasserstoff, Hydroxy, Mercapto, Amino, Cyano, Isocyano, Thiocyanato, Nitro, Carboxy, Carbamoyl, Thiocarbamoyl, Sulfo, Chlorsulfonyl, Halogen, für jeweils gegebenenfalls durch Halogen oder C₁-C₄-Alkoxy substituiertes Alkyl, Alkoxy, Alkylthio, Alkylsulfinyl, Alkylsulfonyl, Alkylamino, Dialkylamino, Alkoxycarbonyl oder Dialkoxy(thio)phosphoryl mit jeweils 1 bis 6 Kohlenstoffatomen in den Alkylgruppen, für jeweils gegebenenfalls durch Halogen substituiertes Alkenyl, Alkenyloxy, Alkenylamino, Alkylidenamino, Alkenyloxycarbonyl, Alkinyl, Alkinyloxy, Alkinylamino oder Alkinyloxycarbonyl mit jeweils 2 bis 6 Kohlenstoffatomen in den Alkenyl-, Alkyliden- oder Alkinylgruppen, für jeweils gegebenenfalls durch Halogen, Cyano, Carboxy, C₁-C₄-Alkyl und/oder C₁-C₄-Alkoxy-carbonyl substituiertes Cycloalkyl, Cycloalkyloxy, Cycloalkylalkyl, Cycloalkylalkoxy, Cycloalkylidenamino, Cycloalkyloxycarbonyl oder Cycloalkylalkoxycarbonyl mit jeweils 3 bis 6 Kohlenstoffatomen in den Cycloalkylgruppen und gegebenenfalls 1 bis 4 Kohlenstoffatomen in den Alkylgruppen, oder für jeweils gegebenenfalls durch Nitro, Cyano, Carboxy, Halogen C₁-C₄-Alkyl, C₁-C₄-Halogenalkyl, C₁-C₄-Alkyloxy, C₁-C₄-Halogenalkyloxy und/oder C₁-C₄-Alkoxy-carbonyl substituiertes Phenyl, Phenyloxy, Phenyl-C₁-C₄-alkyl, Phenyl-C₁-C₄-alkoxy, Phenyloxycarbonyl oder Phenyl-C₁-C₄-alkoxycarbonyl, Oxetanyl, (jeweils gegebenenfalls ganz oder teilweise hydriertes) Pyrrolyl, Pyrazolyl, Imidazolyl, Triazolyl, Furyl, Thienyl, Oxazolyl, Isoxazolyl, Thiazolyl, Isothiazolyl, Oxadiazolyl, Thiadiazolyl, Pyridinyl, Pyrimidinyl, Triazinyl, Pyrazolyl-C₁-C₄-alkyl, Furyl-C₁-C₄-alkyl, Thienyl-C₁-C₄-alkyl, Oxazolyl-C₁-C₄-alkyl, Isoxazol-C₁-C₄-alkyl, Thiazol-C₁-C₄-alkyl, Pyridinyl-C₁-C₄-alkyl, Pyrimidinyl-C₁-C₄-alkyl, Pyrazolylmethoxy, Furylmethoxy, für Perhydropyranylmethoxy oder Pyridylmethoxy steht,
oder die Reste R² und R³ zusammen für eine der nachstehenden Gruppierungen stehen
-Q³-CQ⁴-Q⁵-, -Q³-C(R⁸,R⁹)-Q⁵-, -C(R⁸,R⁹)-Q³-CQ⁴-,
-Q³-C(R⁸,R⁹)-C(R⁸,R⁹)-, -Q³-C(R⁸,R⁹)-C(R⁸,R⁹)-Q⁵-,
-C(R⁸,R⁹)-C(R⁸,R⁹)-CQ⁴-, -Q³-C(R⁸)=C(R⁸)-, -C(R⁸)=C(R⁸)-CQ⁴-,
-Q³-C(R⁸,R⁹)-CQ⁴-, -N(R¹⁰)-C(R⁸;R⁹)-CQ⁴-, -Q³-CQ⁴-C(R⁸,R⁹)-,
-Q³-CQ⁴-N(R¹⁰)-, -Q³-C(R⁸,R⁹)-CQ⁴-N(R¹⁰)-,
-C(R⁸,R⁹)-Q³-CQ⁴-N(R¹⁰)-, -C(R⁸,R⁹)-C(R⁸,R⁹)-N(R¹⁰)-,
-C(R⁸,R⁹)-C(R⁸,R⁹)-CQ⁴-N(R¹⁰)-, -C(R⁸)=C(R⁸)-N(R¹⁰)-,
-C(R⁸)=C(R⁸)-CQ⁴-N(R¹⁰)-, -C(R⁸,R⁹)-CQ⁴-N(R¹⁰)-,
-N(R¹⁰)-C(R⁸;R⁹)-CQ⁴-N(R¹⁰)-, -C(R⁸)=N-N(R¹⁰)-,
-Q³-CQ⁴-C(R⁸,R⁹)-N(R¹⁰)-,
wobei
Q³, Q⁴ und Q⁵ gleich oder verschieden sind und jeweils für Sauerstoff oder Schwefel stehen,
R⁸ und R⁹ gleich oder verschieden sind und einzeln für Wasserstoff, Fluor, Chlor, Brom oder C₁-C₄-Alkyl stehen oder zusammen für C₂-C₅-Alkandiyl stehen, und
R¹⁰ für Wasserstoff, Hydroxy, für gegebenenfalls durch Cyano, Fluor, Chlor, C₁-C₄-Alkoxy, C₁-C₄-Alkyl-carbonyl oder C₁-C₄-Alkoxy-carbonyl substituiertes Alkyl, Alkylcarbonyl, Alkoxycarbonyl oder Alkylsulfonyl mit jeweils 1 bis 6 Kohlenstoffatomen in den Alkylgruppen, für jeweils gegebenenfalls durch Fluor, Chlor oder Brom substituiertes Alkenyl oder Alkinyl mit jeweils 2 bis 6 Kohlenstoffatomen, für jeweils gegebenenfalls durch Fluor, Chlor, Brom oder C₁-C₄-Alkyl substituiertes Cycloalkyl oder Cycloalkylalkyl mit jeweils 3 bis 6 Kohlenstoffatomen in den Cycloalkylgruppen und gegebenenfalls 1 bis 3 Atomen in der Alkylgruppe, für jeweils gegebenenfalls durch Fluor und/oder Chlor substituiertes Alkoxy oder Alkenyloxy mit jeweils bis zu 6 Kohlenstoffatomen, oder für jeweils gegebenenfalls durch Cyano, Fluor, Chlor, C₁-C₄-Alkyl, C₁-C₄-Halogenalkyl, C₁-C₄-Alkoxy oder C₁-C₄-Halogenalkoxy substituiertes Benzyl oder Benzyloxy steht,
R⁴ für Amino, für gegebenenfalls durch Fluor und/oder Chlor substituiertes Alkyl mit 1 bis 4 Kohlenstoffatomen oder für Cycloalkyl mit 3 bis 6 Kohlenstoffatomen steht,
R⁵ für Wasserstoff oder für gegebenenfalls durch Fluor und/oder Chlor substituiertes Alkyl mit 1 bis 4 Kohlenstoffatomen steht,
R⁶ für Wasserstoff oder Alkyl mit 1 bis 4 Kohlenstoffatomen steht, und
R⁷ für Wasserstoff oder für Alkyl, Alkylcarbonyl, Alkoxycarbonyl, Alkylaminocarbonyl, Dialkylaminocarbonyl oder Alkylsulfonyl mit jeweils 1 bis 4 Kohlenstoffatomen in den Alkylgruppen steht, oder
R⁶ und R⁷ zusammen für Alkandiyl mit 2 bis 6 Kohlenstoffatomen oder eine Alkylidenimino-, Cycloalkylalkylidenimino- oder Arylalkylidenimino-Gruppierung mit jeweils bis zu 6 Kohlenstoffatomen im Alkylidenteil und gegebenenfalls 5 oder 6 Kohlenstoffatomen im Cycloalkylteil oder 6 oder 10 Kohlenstoffatomen im Arylteil stehen.

2. Diazacyclohexandi(thi)one gemäß Anspruch 1, **dadurch gekennzeichnet, daß**
Q¹ für Sauerstoff steht,
Q² für Sauerstoff steht,
R¹ für Wasserstoff, Fluor oder Chlor steht,
R² für Cyano, Chlor, Brom, Methyl oder Trifluormethyl steht,
R³ für die nachstehende Gruppierung steht,
-A¹-A²-A³
in welcher
A¹ für eine Einfachbindung, für Sauerstoff, Schwefel, -SO-, -SO₂-, -CO- oder die Gruppierung -N-A⁴- steht, worin A⁴ für Wasserstoff, Hydroxy, Methyl, Ethyl, n- oder i-Propyl, Methoxy, Ethoxy, n- oder i-Propoxy, Methylsulfonyl oder Ethylsulfonyl steht,
A¹ weiterhin für Methylen, Ethan-1,1-diyl, Ethan-1,2-diyl, Propan-1,1-diyl, Propan-1,2-diyl, Propan-1,3-diyl, Ethen-1,2-diyl, Propen-1,2-diyl, Propen-1,3-diyl, Ethin-1,2-diyl, Propin-1,2-diyl oder Propin-1,3-diyl steht,
A² für eine Einfachbindung, für Sauerstoff, Schwefel, -SO-, -SO₂-, -CO- oder die Gruppierung -N-A⁴- steht, worin A⁴ für Wasserstoff, Hydroxy, Methyl, Ethyl, n- oder i-Propyl, Methoxy, Ethoxy, n- oder i-Propoxy, Methylsulfonyl, Ethylsulfonyl, n- oder i-Propylsulfonyl oder Phenylsulfonyl steht,
A² weiterhin für Methylen, Ethan-1,1-diyl, Ethan-1,2-diyl, Propan-1,1-diyl, Propan-1,2-diyl, Propan-1,3-diyl, Ethen-1,2-diyl, Propen-1,2-diyl, Propen-1,3-diyl, Ethin-1,2-diyl, Propin-1,2-diyl oder Propin-1,3-diyl steht,
A³ für Wasserstoff, Hydroxy, Amino, Cyano, Nitro, Carboxy, Carbamoyl, Sulfo, Fluor, Chlor, Brom, für jeweils gegebenenfalls durch Fluor, Chlor, Methoxy oder Ethoxy substituiertes Methyl, Ethyl, n- oder i-Propyl, n-, i-, s- oder t-Butyl, n-, i-, s- oder t-Pentyl, Methoxy, Ethoxy, n- oder i-Propoxy, n-, i-, s- oder t-Butoxy, n-, i-, s- oder t-Pentyloxy, Methylthio, Ethylthio, n- oder i-Propylthio, n-, i-, s- oder t-Butylthio, Methylsulfinyl, Ethylsulfinyl, n- oder i-Propylsulfinyl, Methylsulfonyl, Ethylsulfonyl, n- oder i-Propylsulfonyl, Methylamino, Ethylamino, n- oder i-Propylamino, n-, i-, s- oder t-Butylamino, Dimethylamino, Diethylamino, Methoxycarbonyl, Ethoxycarbonyl, n- oder i-Propoxycarbonyl, Dimethoxyphosphoryl, Diethoxyphosphoryl oder Dipropoxyphosphoryl, Diisopropoxyphosphoryl, für jeweils gegebenenfalls durch Fluor oder Chlor substituiertes Propenyl, Butenyl, Propenyloxy, Butenyloxy, Propenylamino, Butenylamino, Propylidenamino, Butylidenamino, Propenyloxycarbonyl, Butenyloxycarbonyl, Propinyl, Butinyl, Propinyloxy, Butinyloxy, Propinylamino, Butinylamino, Propinyloxycarbonyl oder Butinyloxycarbonyl, für jeweils gegebenenfalls durch Fluor, Chlor, Cyano, Carboxy, Methyl, Ethyl, n- oder i-Propyl, Methoxycarbonyl oder Ethoxycarbonyl substituiertes Cyclopropyl, Cyclobutyl, Cyclopentyl, Cyclohexyl, Cyclopropyloxy, Cyclobutyloxy, Cyclopentyloxy, Cyclohexyloxy, Cyclopropylmethyl, Cyclobutylmethyl, Cyclopentylmethyl, Cyclohexylmethyl, Cyclo-propylmethoxy, Cyclobutylmethoxy, Cyclopentylmethoxy, Cyclohexylmethoxy, Cyclopentylidenamino, Cyclohexylidenamino, Cyclopentyloxycarbonyl, Cyclohexyloxycarbonyl, Cyclopentylmethoxycarbonyl oder Cyclohexylmethoxycarbonyl, oder für jeweils gegebenenfalls durch Nitro, Cyano, Carboxy, Fluor, Chlor, Brom, Methyl, Ethyl, n- oder i-Propyl, Trifluormethyl, Methoxy, Ethoxy, n- oder i-Propoxy, Difluormethoxy, Trifluormethoxy, Methoxycarbonyl und/oder Ethoxycarbonyl substituiertes Phenyl, Phenyloxy, Benzyl, Phenylethyl, Benzyloxy, Phenyloxycarbonyl, Benzyloxycarbonyl, Oxetanyl, (jeweils gegebenenfalls ganz oder teilweise hydriertes) Pyrrolyl, Pyrazolyl, Imidazolyl, Triazolyl, Furyl, Thienyl, Oxazolyl, Isoxazolyl, Thiazolyl, Isothiazolyl, Oxadiazolyl, Thiadiazolyl, Pyridinyl, Pyrimidinyl, Triazinyl, Pyrazolylmethyl, Furylmethyl, Thienylmethyl, Oxazolylmethyl, Isoxazolmethyl, Thiazolmethyl, Pyridinylmethyl, Pyrimidinylmethyl, Pyrazolylmethoxy, Furylmethoxy oder Pyridylmethoxy steht,
oder die Reste R² und R³ zusammen für eine der nachstehenden Gruppierungen stehen
-Q³-CQ⁴-Q⁵-, -Q³-C(R⁸,R⁹)-Q⁵-, -C(R⁸,R⁹)-Q³-CQ⁴-,
-Q³-C(R⁸,R⁹)-C(R⁸,R⁹)-, -Q³-C(R⁸,R⁹)-C(R⁸,R⁹)-Q⁵-,
-C(R⁸,R⁹)-C(R⁸,R⁹)-CQ⁴-, -Q³-C(R⁸)=C(R⁸)-, -C(R⁸)=C(R⁸)-CQ⁴-,
-Q³-C(R⁸,R⁹)-CQ⁴-, -N(R¹⁰)-C(R⁸;R⁹)-CQ⁴-, -Q³-CQ⁴-C(R⁸,R⁹)-,
-Q³-CQ⁴-N(R¹⁰)-, -Q³-C(R⁸,R⁹)-CQ⁴-N(R¹⁰)-,
-C(R⁸,R⁹)-Q³-CQ⁴-N(R¹⁰)-, -C(R⁸,R⁹)-C(R⁸,R⁹)-N(R¹⁰)-,
-C(R⁸,R⁹)-C(R⁸,R⁹)-CQ⁴-N(R¹⁰)-, -C(R⁸)=C(R⁸)-N(R¹⁰)-,
-C(R⁸)=C(R⁸)-CQ⁴-N(R¹⁰)-, -C(R⁸,R⁹)-CQ⁴-N(R¹⁰)-,
-N(R¹⁰)-C(R⁸;R⁹)-CQ⁴-N(R¹⁰)-, -C(R⁸)=N-N(R¹⁰)-,
-Q³-CQ⁴-C(R⁸,R⁹)-N(R¹⁰)-,
wobei
Q³, Q⁴ und Q⁵ gleich oder verschieden sind und jeweils für Sauerstoff oder Schwefel stehen,
R⁸ und R⁹ gleich oder verschieden sind und einzeln für Wasserstoff, Fluor, Chlor, Methyl oder Ethyl stehen oder zusammen für Ethan-1,2-diyl (Dimethylen) stehen, und
R¹⁰ für Wasserstoff, Hydroxy, für gegebenenfalls durch Cyano, Fluor, Chlor, Methoxy, Ethoxy, Acetyl, Propionyl, Methoxycarbonyl oder Ethoxy-carbonyl substituiertes Methyl, Ethyl, n- oder i-Propyl, n-, i-, s oder t-Butyl, für jeweils gegebenenfalls durch Fluor, Chlor oder Brom substituiertes Propenyl, Butenyl, Propinyl oder Butinyl, für jeweils gegebenenfalls durch Fluor, Chlor, Brom, Methyl oder Ethyl substituiertes Cyclopropyl, Cyclobutyl, Cyclopentyl, Cyclohexyl, Cyclopropylmethyl, Cyclobutylmethyl, Cyclopentylmethyl oder Cyclohexylmethyl, für jeweils gegebenenfalls durch Fluor und/oder Chlor substituiertes Methoxy, Ethoxy, n- oder i-Propoxy, n-, i- oder s-Butoxy, Propenyloxy oder Butenyloxy, oder für jeweils gegebenenfalls durch Cyano, Fluor, Chlor, Methyl, Ethyl, Trifluormethyl, Methoxy, Ethoxy, Difluormethoxy oder Trifluormethoxy substituiertes Benzyl oder Benzyloxy steht,
R⁴ für Amino, Methyl, Ethyl, n- oder i-Propyl oder Cyclopropyl steht,
R⁵ für jeweils gegebenenfalls durch Fluor und/oder Chlor substituiertes Methyl oder Ethyl steht,
R⁶ für Wasserstoff, Methyl oder Ethyl steht, und
R⁷ für Wasserstoff, Methyl, Ethyl, Acetyl, Propionyl, Methoxycarbonyl, Ethoxycarbonyl, Methylaminocarbonyl, Ethylaminocarbonyl, Dimethylaminocarbonyl, Methylsulfonyl oder Ethylsulfonyl steht.

3. Verfahren zum Herstellen von Diazacyclohexandi(thi)onen gemäß Anspruch 1 oder 2,
**dadurch gekennzeichnet, daß** man Uracile der allgemeinen Formel (II) 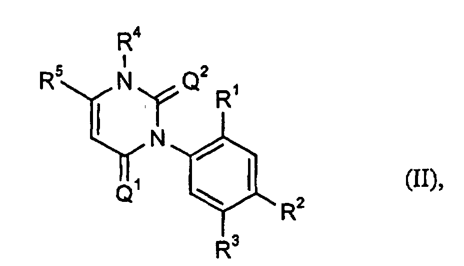 in welcher Q¹, Q², R¹, R², R³, R⁴ und R⁵ die in Anspruch 1 oder 2 angegebenen Bedeutungen haben,
mit Ammoniak gegebenenfalls in Gegenwart eines Verdünnungsmittels umsetzt und gegebenenfalls die so erhaltenen Verbindungen der Formel (I), in welcher R⁶ und R7 für Wasserstoff stehen, nach üblichen Methoden mit Alkylierungs-, Acylierungs- oder Sulfonylierungsmitteln umsetzt.

4. Verfahren zur Bekämpfung von unerwünschten Pflanzen, **dadurch gekennzeichnet, daß** man Diazacylohexandi(thi)one gemäß Anspruch 1 oder 2 auf unerwünschte Pflanzen und/oder ihren Lebensraum einwirken läßt.

5. Verwendung von Diazacyclohexandi(thi)onen gemäß Anspruch 1 oder 2 zur Bekämpfung von unerwünschten Pflanzen.

6. Verfahren zur Herstellung von herbiziden Mitteln, **dadurch gekennzeichnet, daß** man Diazacyclohexandi(thi)one gemäß Anspruch 1 oder 2 mit Streckmitteln und/oder oberflächenaktiven Substanzen vermischt.

7. Herbizide Mittel, **gekennzeichnet durch** einen Gehalt an mindestens einem Diazacyclohexandi(thi)on gemäß Anspruch 1 oder 2.

8. Verbindungen der allgemeinen Formel (II) 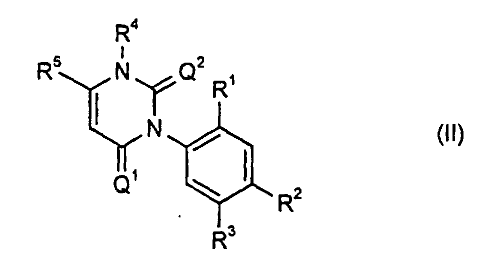 **dadurch gekennzeichnet, daß**
Q¹ für Sauerstoff oder Schwefel steht,
Q² für Sauerstoff oder Schwefel steht,
R¹ für Fluor oder Brom steht,
R² für Cyano oder Methyl steht,
R³ für Amino oder Nitro steht,
R⁴ für Amino, oder für gegebenenfalls durch Fluor und/oder Chlor substituiertes Alkyl mit 1 bis 4 Kohlenstoffatomen oder für Cycloalkyl mit 3 bis 6 Kohlenstoffatomen steht und
R⁵ für Wasserstoff oder für gegebenenfalls durch Fluor und/oder Chlor substituiertes Alkyl mit 1 bis 4 Kohlenstoffatomen steht.

## Claims

1. Diazacyclohexanedi(thio)ones of the general formula (I) 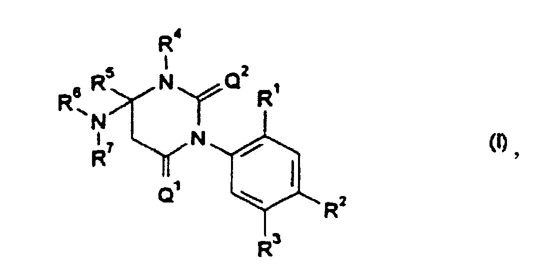 **characterized in that**
Q¹ represents oxygen or sulphur,
Q² represents oxygen or sulphur,
R¹ represents hydrogen, cyano, nitro, fluorine, chlorine or bromine, or represents optionally fluorine- and/or chlorine-substituted alkyl or alkoxy having in each case 1 to 4 carbon atoms,
R² represents hydrogen, cyano, nitro, thiocarbamoyl, fluorine, chlorine or bromine, or represents in each case optionally fluorine- and/or chlorine-substituted alkyl or alkoxy having in each case 1 to 4 carbon atoms,
R³ represents the following grouping
-A¹-A²-A³
in which
A¹ represents a single bond, or represents oxygen, sulphur, -SO-, -SO₂-, -CO- or the grouping -N-A⁴-, wherein A⁴ represents hydrogen, hydroxyl, C₁-C₄-alkyl, C₁-C₄-alkoxy, phenyl, C₁-C₄-alkylsulphonyl or phenylsulphonyl,
A¹ furthermore represents in each case optionally fluorine-, chlorine- or bromine-substituted C₁-C₆-alkanediyl, C₂-C₆-alkenediyl, C₂-C₆-azaalkenediyl, C₂-C₆-alkinediyl, C₃-C₆-cycloalkanediyl, C₃-C₆-cycloalkenediyl or phenylene,
A² represents a single bond, or represents oxygen, sulphur, -SO-, -SO₂-, -CO- or the grouping -N-A⁴-, wherein A⁴ represents hydrogen, hydroxyl, C₁-C₄-alkyl, C₁-C₄-alkoxy, phenyl, C₁-C₄-alkylsulphonyl or phenylsulphonyl,
A² furthermore represents in each case optionally fluorine-, chlorine- or bromine-substituted C₁-C₆-alkanediyl, C₂-C₆-alkenediyl, C₂-C₆-azaalkenediyl, C₂-C₆-alkinediyl, C₃-C₆-cycloalkanediyl, C₃-C₆-cycloalkenediyl or phenylene,
A³ represents hydrogen, hydroxyl, mercapto, amino, cyano, isocyano, thiocyanato, nitro, carboxyl, carbamoyl, thiocarbamoyl, sulpho, chlorosulphonyl or halogen, or represents in each case optionally halogen- or C₁-C₄-alkoxy-substituted alkyl, alkoxy, alkylthio, alkylsulphinyl, alkylsulphonyl, alkylamino, dialkylamino, alkoxycarbonyl or dialkoxy(thio)phosphoryl having in each case 1 to 6 carbon atoms in the alkyl groups, or represents in each case optionally halogen-substituted alkenyl, alkenyloxy, alkenylamino, alkylideneamino, alkenyloxycarbonyl, alkinyl, alkinyloxy, alkinylamino or alkinyloxycarbonyl having in each case 2 to 6 carbon atoms in the alkenyl, alkylidene or alkinyl groups, or represents in each case optionally halo-gen-, cyano-, carboxyl-, C₁-C₄-alkyl- and/or C₁-C₄-alkoxycarbonyl-substituted cyclo-alkyl, cycloalkyloxy, cycloalkylalkyl, cycloalkylalkoxy, cycloalkylideneamino, cycloalkyloxycarbonyl or cycloalkylalkoxycarbonyl having in each case 3 to 6 carbon atoms in the cycloalkyl groups and, where appropriate, 1 to 4 carbon atoms in the alkyl groups, or represents in each case optionally nitro-, cyano-, carboxyl-, halogen, C₁-C₄-alkyl-, C₁-C₄-halogenoalkyl-, C₁-C₄-alkyloxy-, C₁-C₄-halogenoalkyloxy- and/or C₁-C₄-alkoxy-carbonyl-substituted phenyl, phenyloxy, phenyl-C₁-C₄-alkyl, phenyl-C₁-C₄-alkoxy, phenyloxycarbonyl or phenyl-C₁-C₄-alkoxycarbonyl, oxetanyl, (in each case optionally completely or partly hydrogenated) pyrrolyl, pyrazolyl, imidazolyl, triazolyl, furyl, thienyl, oxazolyl, isoxazolyl, thiazolyl, isothiazolyl, oxadiazolyl, thiadiazolyl, pyridinyl, pyrimidinyl, triazinyl, pyrazolyl-C₁-C₄-alkyl, furyl-C₁-C₄-alkyl, thienyl-C₁-C₄-alkyl, oxazolyl-C₁-C₄-alkyl, isoxazole-C₁-C₄-alkyl, thiazole-C₁-C₄-alkyl, pyridinyl-C₁-C₄-alkyl, pyrimidinyl-C₁-C₄-alkyl, pyrazolylmethoxy or furylmethoxy, or represents perhydropyranylmethoxy or pyridylmethoxy,
or the radicals R² and R³ together represent one of the following groupings
-Q³-CQ⁴-Q⁵-, -Q³-C(R⁸,R⁹)-Q⁵-, -C(R⁸,R⁹)-Q³-CQ⁴-,
-Q³-C(R⁸,R⁹)-C(R⁸,R⁹)-, -Q³-C(R⁸,R⁹)-C(R⁸,R⁹)-Q⁵-,
-C(R⁸,R⁹)-C(R⁸,R⁹)-CQ⁴-, -Q³-C(R⁸)=C(R⁸)-, -C(R⁸)=C(R⁸)-CQ⁴-,
-Q³-C(R⁸,R⁹)-CQ⁴-, -N(R¹⁰)-C(R⁸;R⁹)-CQ⁴-, -Q³-CQ⁴-C(R⁸,R⁹)-,
-Q³-CQ⁴-N(R¹⁰)-, -Q³-C(R⁸,R⁹)-CQ⁴-N(R¹⁰)-,
-C(R⁸,R⁹)-Q³-CQ⁴-N(R¹⁰)-, -C(R⁸,R⁹)-C(R⁸,R⁹)-N(R¹⁰)-,
-C(R⁸,R⁹)-C(R⁸,R⁹)-CQ⁴-N(R¹⁰)-, -C(R⁸)=C(R⁸)-N(R¹⁰)-,
-C(R⁸)=C(R⁸)-CQ⁴-N(R¹⁰)-, -C(R⁸,R⁹)-CQ⁴-N(R¹⁰)-,
-N(R¹⁰)-C(R⁸;R⁹)-CQ⁴-N(R¹⁰)-, -C(R⁸)=N-N(R¹⁰)-, or
-Q³-CQ⁴-C(R⁸,R⁹)-N(R¹⁰)-,
wherein
Q³, Q⁴ and Q⁵ are identical or different and in each case represent oxygen or sulphur,
R⁸ and R⁹ are identical or different and individually represent hydrogen, fluorine, chlorine, bromine or C₁-C₄-alkyl, or together represent C₂-C₅-alkanediyl and
R¹⁰ represents hydrogen or hydroxyl, or represents optionally cyano-, fluorine-, chlorine-, C₁-C₄-alkoxy-, C₁-C₄-alkyl-carbonyl- or C₁-C₄-alkoxycarbonyl-substituted alkyl, alkylcarbonyl, alkoxycarbonyl or alkylsulphonyl having in each case 1 to 6 carbon atoms in the alkyl groups, or represents in each case optionally fluorine-, chlorine- or bromine-substituted alkenyl or alkinyl having in each case 2 to 6 carbon atoms, or represents in each case optionally fluorine-, chlorine-, bromine- or C₁-C₄-alkyl-substituted cycloalkyl or cycloalkylalkyl having in each case 3 to 6 carbon atoms in the cycloalkyl groups and, where appropriate, 1 to 3 carbon atoms in the alkyl group, or represents in each case optionally fluorine- and/or chlorine-substituted alkoxy or alkenyloxy having in each case up to 6 carbon atoms, or represents in each case optionally cyano-, fluorine-, chlorine-, C₁-C₄-alkyl-, C₁-C₄-halogenoalkyl-, C₁-C₄-alkoxy- or C₁-C₄-halogenoalkoxy-substituted benzyl or benzyloxy,
R⁴ represents amino, or represents optionally fluorine- and/or chlorine-substituted alkyl having 1 to 4 carbon atoms, or represents cycloalkyl having 3 to 6 carbon atoms,
R⁵ represents hydrogen, or represents optionally fluorine- and/or chlorine-substituted alkyl having 1 to 4 carbon atoms,
R⁶ represents hydrogen or alkyl having 1 to 4 carbon atoms and
R⁷ represents hydrogen, or represents alkyl, alkylcarbonyl, alkoxycarbonyl, alkylaminocarbonyl, dialkylaminocarbonyl or alkylsulphonyl having in each case 1 to 4 carbon atoms in the alkyl groups, or
R⁶ and R⁷ together represent alkanediyl having 2 to 6 carbon atoms or an alkylideneimino, cycloalkylalkylideneimino or arylalkylideneimino grouping having in each case up to 6 carbon atoms in the alkylidene part and, where appropriate, 5 or 6 carbon atoms in the cycloalkyl part or 6 or 10 carbon atoms in the aryl part.

2. Diazacyclohexanedi(thi)ones according to Claim 1, **characterized in that**
Q¹ represents oxygen,
Q² represents oxygen,
R¹ represents hydrogen, fluorine or chlorine,
R² represents cyano, chlorine, bromine, methyl or trifluoromethyl,
R³ represents the following grouping
-A¹-A²-A³
in which
A¹ represents a single bond, or represents oxygen, sulphur, -SO-, -SO₂-, -CO- or the grouping -N-A⁴-, wherein A⁴ represents hydrogen, hydroxyl, methyl, ethyl, n- or i-propyl, methoxy, ethoxy, n- or i-propoxy, methylsulphonyl or ethylsulphonyl,
A¹ furthermore represents methylene, ethane-1,1-diyl, ethane-1,2-diyl, propane-1,1-diyl, propane-1,2-diyl, propane-1,3-diyl, ethene-1,2-diyl, propene-1,2-diyl, propene-1,3-diyl, ethine-1,2-diyl, propine-1,2-diyl or propine-1,3-diyl,
A² represents a single bond, or represents oxygen, sulphur, -SO-, -SO₂-, -CO- or the grouping -N-A⁴-, wherein A⁴ represents hydrogen, hydroxyl, methyl, ethyl, n- or i-propyl, methoxy, ethoxy, n- or i-propoxy, methylsulphonyl, ethylsulphonyl, n- or i-propylsulphonyl or phenylsulphonyl,
A² furthermore represents methylene, ethane-1,1-diyl, ethane-1,2-diyl, propane-1,1-diyl, propane-1,2-diyl, propane-1,3-diyl, ethene-1,2-diyl, propene-1,2-diyl, propene-1,3-diyl, ethine-1,2-diyl, propine-1,2-diyl or propine-1,3-diyl,
A³ represents hydrogen, hydroxyl, amino, cyano, nitro, carboxyl, carbamoyl, sulpho, fluorine, chlorine or bromine, or represents in each case optionally fluorine-, chlorine-, methoxy- or ethoxy-substituted methyl, ethyl, n- or i-propyl, n-, i-, s- or t-butyl, n-, i-, s- or t-pentyl, methoxy, ethoxy, n- or i-propoxy, n-, i-, s- or t-butoxy, n-, i-, s- or t- pentyloxy, methylthio, ethylthio, n- or i-propylthio, n-, i-, s- or t-butylthio, methylsulphinyl, ethylsulphinyl, n- or i-propylsulphinyl, methylsulphonyl, ethylsulphonyl, n- or i-propylsulphonyl, methylamino, ethylamino, n- or i-propylamino, n-, i-, s- or t-butylamino, dimethylamino, diethylamino, methoxycarbonyl, ethoxycarbonyl, n- or i-propoxycarbonyl, dimethoxyphosphoryl, diethoxyphosphoryl or dipropoxyphosphoryl or diisopropoxyphosphoryl, or represents in each case optionally fluorine- or chlorine-substituted propenyl, butenyl, propenyloxy, butenyloxy, propenylamino, butenylamino, propylideneamino, butylideneamino, propenyloxycarbonyl, butenyloxycarbonyl, propinyl, butinyl, propinyloxy, butinyloxy, propinylamino, butinylamino, propinyloxycarbonyl or butinyloxycarbonyl, or represents in each case optionally fluorine-, chlorine-, cyano-, carboxyl-, methyl-, ethyl-, n- or i-propyl-, methoxycarbonyl- or ethoxycarbonyl-substituted cyclopropyl, cyclobutyl, cyclopentyl, cyclohexyl, cyclopropyloxy, cyclobutyloxy, cyclopentyloxy, cyclohexyloxy, cyclopropylmethyl, cyclobutylmethyl, cyclopentylmethyl, cyclohexylmethyl, cyclopropylmethoxy, cyclobutylmethoxy, cyclopentylmethoxy, cyclohexylmethoxy, cyclopentylideneamino, cyclohexylideneamino, cyclopentyloxycarbonyl, cyclohexyloxycarbonyl, cyclopentylmethoxycarbonyl or cyclohexylmethoxycarbonyl, or represents in each case optionally nitro-, cyano-, carboxyl-, fluorine-, chlorine-, bromine-, methyl-, ethyl-, n-or i-propyl-, trifluoromethyl-, methoxy-, ethoxy-, n- or i-propoxy-, difluoromethoxy-, trifluoromethoxy-, methoxycarbonyl- and/or ethoxycarbonyl-substituted phenyl, phenyloxy, benzyl, phenylethyl, benzyloxy, phenyloxycarbonyl, benzyloxycarbonyl, oxetanyl, (in each case optionally completely or partly hydrogenated) pyrrolyl, pyrazolyl, imidazolyl, triazolyl, furyl, thienyl, oxazolyl, isoxazolyl, thiazolyl, isothiazolyl, oxadiazolyl, thiadiazolyl, pyridinyl, pyrimidinyl, triazinyl, pyrazolylmethyl, furylmethyl, thienylmethyl, oxazolylmethyl, isoxazolemethyl, thiazolemethyl, pyridinylmethyl, pyrimidinylmethyl, pyrazolylmethoxy, furylmethoxy or pyridylmethoxy,
or the radicals R² and R³ together represent one of the following groupings
-Q³-CQ⁴-Q⁵-, -Q³-C(R⁸,R⁹)-Q⁵-, -C(R⁸,R⁹)-Q³-CQ⁴-,
-Q³-C(R⁸,R⁹)-C(R⁸,R⁹)-, -Q³-C(R⁸,R⁹)-C(R⁸,R⁹)-Q⁵-,
-C(R⁸,R⁹)-C(R⁸,R⁹)-CQ⁴-, -Q³-C(R⁸)=C(R⁸)-, -C(R⁸)=C(R⁸)-CQ⁴-,
-Q³-C(R⁸,R⁹)-CQ⁴-, -N(R¹⁰)-C(R⁸;R⁹)-CQ⁴-, -Q³-CQ⁴-C(R⁸,R⁹)-,
-Q³-CQ⁴-N(R¹⁰)-, -Q³-C(R⁸,R⁹)-CQ⁴-N(R¹⁰)-,
-C(R⁸,R⁹)-Q³-CQ⁴-N(R¹⁰)-, -C(R⁸,R⁹)-C(R⁸,R⁹)-N(R¹⁰)-,
-C(R⁸,R⁹)-C(R⁸,R⁹)-CQ⁴-N(R¹⁰)-, -C(R⁸)=C(R⁸)-N(R¹⁰)-,
-C(R⁸)=C(R⁸)-CQ⁴-N(R¹⁰)-, -C(R⁸,R⁹)-CQ⁴-N(R¹⁰)-,
-N(R¹⁰)-C(R⁸;R⁹)-CQ⁴-N(R¹⁰)-, -C(R⁸)=N-N(R¹⁰)-, or
-Q³-CQ⁴-C(R⁸,R⁹)-N(R¹⁰)-,
wherein
Q³, Q⁴ and Q⁵ are identical or different and in each , case represent oxygen or sulphur,
R⁸ and R⁹ are identical or different and individually represent hydrogen, fluorine, chlorine, methyl or ethyl, or together represent ethane-1,2-diyl (dimethylene), and
R¹⁰ represents hydrogen or hydroxyl, or represents optionally cyano-, fluorine-, chlorine-, methoxy-, ethoxy-, acetyl-, propionyl-, methoxycarbonyl- or ethoxy-carbonyl-substituted methyl, ethyl, n- or i-propyl or n-, i-, s- or t-butyl, or represents in each case optionally fluorine-, chlorine- or bromine-substituted propenyl, butenyl, propinyl or butinyl, or represents in each case optionally fluorine-, chlorine-, bromine-, methyl- or ethyl-substituted cyclopropyl, cyclobutyl, cyclopentyl, cyclohexyl, cyclopropylmethyl, cyclobutylmethyl, cyclopentylmethyl or cyclohexylmethyl, or represents in each case optionally fluorine- and/or chlorine-substituted methoxy, ethoxy, n- or i-propoxy, n-, i- or s-butoxy, propenyloxy or butenyloxy, or represents in each case optionally cyano-, fluorine-, chlorine-, methyl-, ethyl-, trifluoromethyl-, methoxy-, ethoxy-, difluoromethoxy- or trifluoromethoxy-substituted benzyl or benzyloxy,
R⁴ represents amino, methyl, ethyl, n- or i-propyl or cyclopropyl,
R⁵ represents in each case optionally fluorine- and/or chlorine-substituted methyl or ethyl,
R⁶ represents hydrogen, methyl or ethyl and
R⁷ represents hydrogen, methyl, ethyl, acetyl, propionyl, methoxycarbonyl, ethoxycarbonyl, methylaminocarbonyl, ethylaminocarbonyl, dimethylaminocarbonyl, methylsulphonyl or ethylsulphonyl.

3. Process for the preparation of new diazacyclohexanedi(thi)ones according to Claim 1 or 2,
**characterized in that** uracils of the general formula (II) 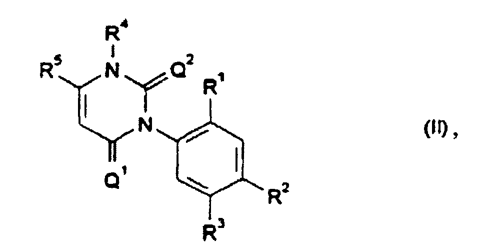 in which Q¹, Q², R¹, R², R³, R⁴ and R⁵ have the meanings given in Claim 1 or 2,
are reacted with ammonia, if appropriate in the presence of a diluent, and, if appropriate, the compounds thus obtained of the formula (I) in which R⁶ and R⁷ represent hydrogen are reacted with alkylating, acylating or sulphonylating agents by customary methods.

4. Method of controlling unwanted plants, **characterized in that** diazacylcohexanedi(thi)ones according to Claim 1 or 2 are allowed to act on unwanted plants and/or their environment.

5. Use of diazacyclohexanedi(thi)ones according to Claim 1 or 2 for controlling undesirable plants.

6. Process for the production of herbicidal compositions, **characterized in that** diazacyclohexanedi(thi)ones according to Claim 1 or 2 are mixed with extenders and/or surfactants.

7. Herbicidal compositions, **characterized by** a content of at least one diazacyclohexanedi(thi)one according to Claim 1 or 2.

8. Compounds of the general formula (II) 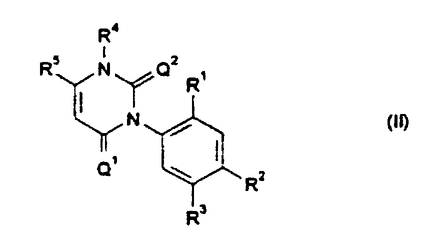 **characterized in that**
Q¹ represents oxygen or sulphur,
Q² represents oxygen or sulphur,
R¹ represents fluorine or bromine,
R² represents cyano or methyl,
R³ represents amino or nitro,
R⁴ represents amino, or represents optionally fluorine- and/or chlorine-substituted alkyl having 1 to 4 carbon atoms, or represents cycloalkyl having 3 to 6 carbon atoms, and
R⁵ represents hydrogen, or represents optionally fluorine- and/or chlorine-substituted alkyl having 1 to 4 carbon atoms.

## Revendications

1. Diazacyclohexanedi(thi)ones de la formule générale (I) : 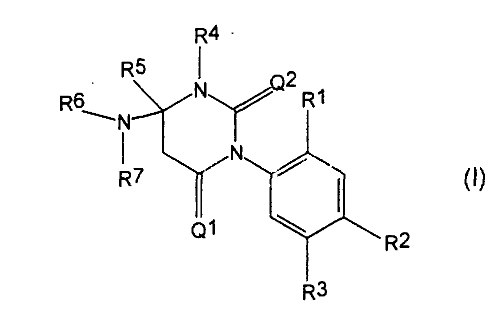 **caractérisés en ce que** :
Q¹ représente l'atome d'oxygène ou de soufre ;
Q² représente l'atome d'oxygène ou de soufre ;
R¹ représente l'atome d'hydrogène, le radical nitro, l'atome de fluor, chlore, brome ou un radical alcoyle ou alcoxy ayant chaque fois 1 à 4 atomes de carbone, le cas échéant substitué par l'atome de fluor et/ou de chlore ;
R² représente l'atome d'hydrogène, le radical cyano, nitro, thiocarbamoyle, l'atome de fluor, chlore, brome ou un radical alcoyle ou alcoxy ayant chaque fois 1 à 4 atomes de carbone, le cas échéant substitué par l'atome de fluor et/ou de chlore ;
R³ représente le groupement suivant :
-A¹-A²-A³
dans lequel :
A¹ représente une simple liaison, l'atome d'oxygène, de soufre, le radical -SO-, -SO₂-, -CO- ou le groupement -N-A⁴-, où A⁴ représente l'atome d'hydrogène, le radical hydroxyle, un radical alcoyle en C₁-C₄, alcoxy en C₁-C₄, phényle, alcoylsulfonyle en C₁-C₄ ou phénylsulfonyle ;
A¹ représente d'autre part, un radical alcanediyle en C₁-C₆, alcènediyle en C₂-C₆, azaalcènediyle en C₂-C₆, alcynediyle en C₂-C₆, cycloalcanediyle en C₃-C₆, cycloalcènediyle en C₃-C₆ ou phénylène, chaque fois le cas échéant substitué par l'atome de fluor, de chlore ou de brome ;
A² représente une simple liaison, l'atome d'oxygène, de soufre, le radical -SO-, -SO₂-, -CO- ou le groupement -N-A⁴-, où A⁴ représente l'atome d'hydrogène, le radical hydroxyle, un radical alcoyle en C₁-C₄, alcoxy en C₁-C₄, phényle, alcoylsulfonyle en C₁-C₄ ou phénylsulfonyle ;
A² représente d'autre part, un radical alcanediyle en C₁-C₆, alcènediyle en C₂-C₆, azaalcènediyle en C₂-C₆, alcynediyle en C₂-C₆, cycloalcanediyle en C₃-C₆, cycloalcènediyle en C₃-C₆ ou phénylène, chaque fois le cas échéant substitué par l'atome de fluor, de chlore ou de brome ;
A³ représente l'atome d'hydrogène, le radical hydroxyle, mercapto, amino, cyano, isocyano, thiocyanato, nitro, carboxyle, carbamoyle, thiocarbamoyle, sulfo, chlorosulfonyle, un atome d'halogène, un radical alcoyle, alcoxy, alcoylthio, alcoylsulfinyle, alcoylsulfonyle, alcoylamino, dialcoylamino, alcoxycarbonyle ou dialcoxy(thio)phosphoryle ayant chaque fois 1 à 6 atomes de carbone dans les radicaux alcoyle, chaque fois le cas échéant substitué par un atome d'halogène ou un radical alcoxy en C₁-C₄, représente un radical alcényle, alcényloxy, alcénylamino, alcoylidèneamino, alcényloxycarbonyle, alcynyle, alcynyloxy, alcynylamino ou alcynyloxycarbonyle ayant chaque fois 2 à 6 atomes de carbone dans les radicaux alcényle, alcoylidène ou alcynyle, chaque fois le cas échéant substitué par un atome d'halogène, représente un radical cycloalcoyle, cycloalcoxy, cycloalcoylalcoyle, cycloalcoylalcoxy, cycloalcoylidèneamino, cycloalcoxycarbonyle ou cycloalcoylalcoxycarbonyle ayant chaque fois 3 à 6 atomes de carbone dans les radicaux cycloalcoyle et le cas échéant, 1 à 4 atomes de carbone dans les radicaux alcoyle, chaque fois le cas échéant substitué par un atome d'halogène, le radical cyano, carboxyle, un radical alcoyle en C₁-C₄ et/ou (alcoxy en C₁-C₄) carbonyle, ou représente un radical phényle, phényloxy, phényl(alcoyle en C₁-C₄), phényl(alcoxy en C₁-C₄), phényloxycarbonyle ou phényl(alcoxy en C₁-C₄) carbonyle, oxétannyle, pyrrolyle, pyrazolyle, imidazolyle, triazolyle, furyle, thiényle, oxazolyle, isoxazolyle, thiazolyle, isothiazolyle, oxadiazolyle, thiadiazolyle, pyridinyle, pyrimidinyle, triazinyle, pyrazolyl(alcoyle en C₁-C₄), furyl(alcoyle en C₁-C₄), thiényl(alcoyle en C₁-C₄), oxazolyl(alcoyle en C₁-C₄), isoxazolyl(alcoyle en C₁-C₄), thiazol(alcoyle en C₁-C₄), pyridinyl(alcoyle en C₁-C₄), pyrimidinyl(alcoyle en C₁-C₄), pyrazolylméthoxy, furylméthoxy, (chaque fois le cas échéant, complètement ou partiellement hydrogéné), chaque fois le cas échéant substitué par le radical nitro, cyano, carboxyle, un atome d'halogène, un radical alcoyle en C₁-C₄, halogénoalcoyle en C₁-C₄, alcoxy en C₁-C₄, halogénoalcoxy en C₁-C₄ et/ou (alcoxy en C₁-C₄)carbonyle, représente le radical perhydropyrannylméthoxy ou pyridylméthoxy ;
ou les restes R² et R³ représentent ensemble l'un des groupements suivants :
-Q³-CQ⁴-Q⁵-, -Q³-C(R⁸,R⁹)-Q⁵-, -C (R⁸,R⁹)-Q³-CQ⁴-, -Q³-C(R⁸,R⁹)-C(R⁸,R⁹)-, -Q³-C(R⁸,R⁹)-C(R⁸,R⁹)-Q⁵-, -C(R⁸,R⁹)-C(R⁸,R⁹)-CQ⁴-, -Q³-C(R⁸)=C(R⁸)-, -C(R⁸)=C(R⁸)-CQ⁴-, -Q³-C(R⁸,R⁹)-CQ⁴-, -N(R¹⁰)-C(R⁸,R⁹)-CQ⁴-, -Q³-CQ⁴-C(R⁸,R⁹)-, -Q³-CQ⁴-N(R¹⁰)-, -Q³-C(R⁸,R⁹)-CQ⁴-N(R¹⁰)-, -C(R⁸,R⁹)-Q³-CQ⁴-N(R¹⁰)-, -C(R⁸,R⁹)-C(R⁸,R⁹)-N(R¹⁰)-, -C(R⁸,R⁹)-C(R⁸,R⁹)-CQ⁴-N(R¹⁰)-, -C(R⁸)=C(R⁸)-N(R¹⁰)-, -C(R⁸)=C(R⁸)-CQ⁴-N(R¹⁰)-, -C(R⁸,R⁹)-CQ⁴-N(R¹⁰)-, -N(R¹⁰)-C(R⁸,R⁹)-CQ⁴-N(R¹⁰)-, -C(R⁸)=N-N(R¹⁰)-, -Q³-CQ⁴-C(R⁸,R⁹)-N(R¹⁰)-,
où
Q³, Q⁴ et Q⁵ sont identiques ou différents et représentent chacun l'atome d'oxygène ou de soufre,
R⁸ et R⁹ sont identiques ou différents et représentent chacun l'atome d'hydrogène, de fluor, de chlore, de brome ou un radical alcoyle en C₁-C₄ ou ensemble, un radical alcanediyle en C₂-C₅, et
R¹⁰ représente l'atome d'hydrogène, le radical hydroxyle, un radical alcoyle, alcoylcarbonyle, alcoxycarbonyle ou alcoylsulfonyle, ayant chaque fois 1 à 6 atomes de carbone dans les radicaux alcoyle, chaque fois le cas échéant substitué par le radical cyano, l'atome de fluor, de chlore, un radical alcoxy en C₁-C₄, (alcoyl en C₁-C₄)carbonyle ou (alcoxy en C₁-C₄) carbonyle, représente un radical alcényle ou alcynyle ayant chaque fois 2 à 6 atomes de carbone, chaque fois le cas échéant substitué par l'atome de fluor, de chlore ou de brome, représente un radical cycloalcoyle ou cycloalcoylalcoyle ayant chaque fois 3 à 6 atomes de carbone dans le radical cycloalcoyle et le cas échéant, 1 à 3 atomes de carbone dans le radical alcoyle, chaque fois le cas échéant substitué par l'atome de fluor, de chlore, de brome ou un radical alcoyle en C₁-C₄, représente un radical alcoxy ou alcényloxy ayant chaque fois jusqu'à 6 atomes de carbone, chaque fois le cas échéant substitué par l'atome de fluor et/ou de chlore, ou représente le radical benzyle ou benzyloxy, chaque fois le cas échéant substitué par le radical cyano, l'atome de fluor, de chlore, un radical alcoyle en C₁-C₄, halogénoalcoyle en C₁-C₄, alcoxy en C₁-C₄ ou halogénoalcoxy en C₁-C₄ ;
R⁴ représente le radical amino, un radical alcoyle ayant 1 à 4 atomes de carbone le cas échéant substitué par l'atome de fluor et/ou de chlore ou représente un radical cycloalcoyle ayant 3 à 6 atomes de carbone ;
R⁵ représente l'atome d'hydrogène ou un radical alcoyle ayant 1 à 4 atomes de carbone le cas échéant substitué par l'atome de fluor et/ou de chlore ;
R⁶ représente l'atome d'hydrogène ou un radical alcoyle ayant 1 à 4 atomes de carbone ;
R⁷ représente l'atome d'hydrogène ou un radical alcoyle, alcoylcarbonyle, alcoxycarbonyle, alcoylaminocarbonyle, dialcoylaminocarbonyle ou alcoylsulfonyle ayant chaque 1 à 4 atomes de carbone dans le radical alcoyle, ou
R⁶ et R⁷ représentent ensemble un radical alcanediyle ayant 2 à 6 atomes de carbone ou un groupement alcoylidènimino, cycloalcoylalcoylidènimino ou arylalcoylidènimino ayant chaque fois, jusqu'à 6 atomes de carbone dans la partie alcoylidène et le cas échéant, 5 ou 6 atomes de carbone dans la partie cycloalcoyle ou 6 à 10 atomes de carbone dans la partie aryle.

2. Diazacyclohexanedi(thi)ones suivant la revendication 1, **caractérisées en ce que** :
Q¹ représente l'atome d'oxygène ;
Q² représente l'atome d'oxygène ;
R¹ représente l'atome d'hydrogène, de fluor ou de chlore ;
R² représente le radical cyano, l'atome de chlore, de brome, le radical méthyle ou trifluorométhyle ;
R³ représente le groupement suivant :
-A¹-A²-A³
dans lequel :
A¹ représente une simple liaison, l'atome d'oxygène, de soufre, le radical -SO-, -SO₂-, -CO- ou le groupement -N-A⁴-, où A⁴ représente l'atome d'hydrogène, le radical hydroxyle, méthyle, éthyle, n- ou i-propyle, méthoxy, éthoxy, n- ou i-propoxy, méthylsulfonyle ou éthylsulfonyle ;
A¹ représente d'autre part, le radical méthylène, éthan-1,1-diyle, éthan-1,2-diyle, propan-1,1-diyle, propan-1,2-diyle, propan-1,2-diyle, propan-1,3-diyle, éthèn-1,2-diyle, propèn-1,2-diyle, propèn-1,3-diyle, éthyn-1,2-diyle, propyn-1,2-diyle ou propyn-1,3-diyle ;
A² représente une simple liaison, l'atome d'oxygène, de soufre, le radical -SO-, -SO₂-, -CO- ou le groupement -N-A⁴-, où A⁴ représente l'atome d'hydrogène, le radical hydroxyle, le radical méthyle, éthyle, n- ou i-propyle, méthoxy, éthoxy, n- ou i-propoxy, méthylsulfonyle, éthylsulfonyle, n- ou i-propylsulfonyle ou phénylsulfonyle ;
A² représente d'autre part, le radical méthylène, éthan-1,1-diyle, éthan-1,2-diyle, propan-1,1-diyle, propan-1,2-diyle, propan-1,3-diyle, éthèn-1,2-diyle, propèn-1,2-diyle, propèn-1,3-diyle, éthyn-1,2-diyle, propyn-1,2-diyle ou propyn-1,3-diyle, ;
A³ représente l'atome d'hydrogène, le radical hydroxyle, amino, cyano, nitro, carboxy, carbamoyle, sulfo, l'atome de fluor, de chlore, de brome, méthyle, éthyle, n- ou i-propyle, n-, i-, s- ou t-butyle, n-, i-, s- ou t-pentyle, méthoxy, éthoxy, n- ou i-propoxy, n-, i-, s- ou t-butoxy, n-, i-, s- ou t-pentoxy, méthylthio, éthylthio, n- ou i-propylthio, n-, i-, s- ou t-butylthio, méthylsulfinyle, éthylsulfinyle, n- ou i-propylsulfinyle, méthylsulfonyle, éthylsulfonyle, n- ou i-propylsulfonyle, méthylamino, éthylamino, n- ou i-propylamino, n-, i-, s- ou t-butylamino, diméthylamino, diéthylamino, méthoxycarbonyle, éthoxycarbonyle, n- ou i-propoxycarbonyle, diméthoxyphosphoryle, diéthoxyphosphoryle ou dipropoxyphosphoryle, chaque fois le cas échéant substitué par l'atome de fluor, de chlore, le radical méthoxy ou éthoxy, représente le radical propényle, butényle, propényloxy, butényloxy, propénylamino, buténylamino, propylidènamino, butylidènamino, propényloxycarbonyle, butényloxycarbonyle, propynyle, butynyle, propynyloxy, butynyloxy, propynylamino, butynylamino, propynyloxycarbonyle ou butynyloxycarbonyle, chaque fois le cas échéant substitué par l'atome de fluor ou de chlore, représente le radical cyclopropyle, cyclobutyle, cyclopentyle, cyclohexyle, cyclopropyloxy, cyclobutyloxy, cyclopentyloxy, cyclohexyloxy, cyclopropylméthyle, cyclobutylméthyle, cyclopentylméthyle, cyclohexylméthyle, cyclopropylméthoxy, cyclobutylméthoxy, cyclopentylméthoxy, cyclohexylméthoxy, cyclopentylidènamino, cyclohexylidènamino, cyclopentyloxycarbonyle, cyclohexyloxycarbonyle, cyclopentylméthoxycarbonyle ou cyclohexylméthoxycarbonyle, chaque fois le cas échéant substitué par l'atome de fluor, de chlore, le radical cyano, carboxy, méthyle, éthyle, n- ou i-propyle, méthoxycarbonyle ou éthoxycarbonyle ou représente le radical phényle, phényloxy, benzyle, phényléthyle, benzyloxy, phényloxycarbonyle, benzyloxycarbonyle, oxétannyle, pyrrolyle, pyrazolyle, imidazolyle, triazolyle, furyle, thiényle, oxazolyle, isoxazolyle, thiazolyle, isothiazolyle, oxadiazolyle, thiadiazolyle, pyridinyle, pyrimidinyle, triazinyle, pyrazolylméthyle, furylméthyle, thiénylméthyle, oxazolylméthyle, isoxazolylméthyle, thiazolméthyle, pyridinylméthyle, pyrimidinylméthyle, pyrazolylméthoxy, furylméthoxy ou pyridylméthoxy, (chaque fois le cas échéant, complètement ou partiellement hydrogéné), chaque fois le cas échéant substitué par le radical nitro, cyano, carboxy, l'atome de fluor, de chlore, de brome, le radical méthyle, éthyle, n- ou i-propyle, trifluorométhyle, méthoxy, éthoxy, n- ou i-propoxy, difluorométhoxy, trifluorométhoxy, méthoxycarbonyle et/ou éthoxycarbonyle ;
ou les restes R² et R³ représentent ensemble l'un des groupements suivants :
-Q³-CQ⁴-Q⁵-, -Q³-C(R⁸,R⁹)-Q⁵-, -C(R⁸,R⁹)-Q³-CQ⁴-, -Q³-C(R⁸,R⁹)-C(R⁸,R⁹)-, -Q³-C(R⁸,R⁹)-C(R⁸,R⁹)-Q⁵-, -C(R⁸,R⁹)-C(R⁸,R⁹)-CQ⁴-, -Q³-C(R⁸)=C(R⁸)-, -C(R⁸)=C(R⁸)-CQ⁴-, -Q³-C(R⁸,R⁹)-CQ⁴-, -N(R¹⁰)-C(R⁸,R⁹)-CQ⁴-, -Q³-CQ⁴-C(R⁸,R⁹)-, -Q³-CQ⁴-N(R¹⁰)-, -Q³-C(R⁸,R⁹)-CQ⁴-N(R¹⁰)-, -C(R⁸,R⁹)-Q³-CQ⁴-N(R¹⁰)-, -C(R⁸,R⁹)-C(R⁸,R⁹)-N(R¹⁰)-, -C(R⁸,R⁹)-C(R⁸,R⁹)-CQ⁴-N(R¹⁰)-, -C(R⁸)=C(R⁸)-N(R¹⁰)-, -C(R⁸)=C(R⁸)-CQ⁴-N(R¹⁰)-, -C(R⁸,R⁹)-CQ⁴-N(R¹⁰)-, -N(R¹⁰)-C(R⁸,R⁹)-CQ⁴-N(R¹⁰)-, -C(R⁸)=N-N(R¹⁰)-, -Q³-CQ⁴-C(R⁸,R⁹)-N(R¹⁰)-,
où
Q³, Q⁴ et Q⁵ sont identiques ou différents et représentent chacun l'atome d'oxygène ou de soufre,
R⁸ et R⁹ sont identiques ou différents et représentent chacun l'atome d'hydrogène, de fluor, de chlore, le radical méthyle ou éthyle ou ensemble, le radical éthan-1,2-diyle (diméthylène), et
R¹⁰ représente l'atome d'hydrogène, le radical hydroxyle, le radical méthyle, éthyle, n- ou i-propyle, n-, i-, s- ou t-butyle, chaque fois le cas échéant substitué par le radical cyano, l'atome de fluor, de chlore, le radical méthoxy, éthoxy, acétyle, propionyle, méthoxycarbonyle ou éthoxycarbonyle, représente un radical propényle, butényle, propynyle ou butynyle, chaque fois le cas échéant substitué par l'atome de fluor, de chlore ou de brome, représente le radical cyclopropyle, cyclobutyle, cyclopentyle, cyclohexyle, cyclopropylméthyle, cyclobutylméthyle, cyclopentylméthyle ou cyclohexylméthyle, chaque fois le cas échéant substitué par l'atome de fluor, de chlore, de brome, le radical méthyle ou éthyle, représente le radical méthoxy, éthoxy, n- ou i-propoxy, n-, i- ou s-butoxy, propényloxy ou butényloxy, chaque fois le cas échéant substitué par l'atome de fluor et/ou de chlore, ou représente le radical benzyle ou benzyloxy, chaque fois le cas échéant substitué par le radical cyano, l'atome de fluor, de chlore, le radical méthyle, éthyle, trifluorométhyle, méthoxy, éthoxy, difluorométhoxy ou trifluorométhoxy ;
R⁴ représente le radical amino, le radical méthyle, éthyle, n- ou i-propyle ou cyclopropyle ;
R⁵ représente le radical méthyle ou éthyle, chaque fois le cas échéant substitué par l'atome de fluor et/ou de chlore ;
R⁶ représente l'atome d'hydrogène, le radical méthyle ou éthyle ;
R⁷ représente l'atome d'hydrogène, le radical le radical méthyle, éthyle, acétyle, propionyle, méthoxycarbonyle, éthoxycarbonyle, méthylaminocarbonyle, éthylaminocarbonyle, diméthylaminocarbonyle, méthylsulfonyle ou éthylsulfonyle.

3. Procédé de préparation des diazacyclohexanedi(thi)ones suivant la revendication 1 ou 2, **caractérisé en ce que** l'on fait réagir des uraciles de la formule générale (II) : 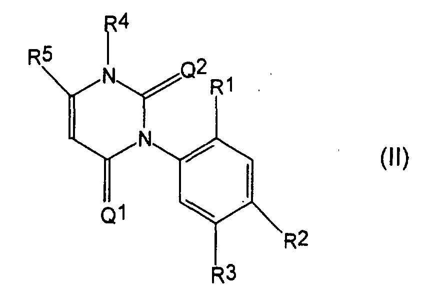 dans laquelle Q¹, Q², R¹, R², R³, R⁴ et R⁵ ont les significations données dans la revendication 1 ou 2,
avec de l'ammoniac, le cas échéant en présence d'un agent de dilution et le cas échéant, les composés ainsi obtenus de la formule (I), dans laquelle R⁶ et R⁷ représentent l'atome d'hydrogène, sont mis à réagir selon les procédés usuels, avec des agents d'alcoylation, d'acylation ou de sulfonylation.

4. Procédé pour lutter contre les plantes non désirées, **caractérisé en ce que** l'on fait agir les diazacyclohexanedi(thi)ones suivant la revendication 1 ou 2, sur les plantes non désirées et/ou leur biotope.

5. Utilisation des diazacyclohexanedi(thi)ones suivant la revendication 1 ou 2, pour lutter contre les plantes non désirées.

6. Procédé de préparation d'un agent herbicide, **caractérisé en ce que** l'on mélanges les diazacyclohexanedi(thi)ones suivant la revendication 1
ou 2, avec un diluant et/ou des substances tensioactives.

7. Herbicide, **caractérisé par** une teneur en au moins une diazacyclohexanedi(thi)one suivant la revendication 1 ou 2.

8. Composés de la formule générale (II) : 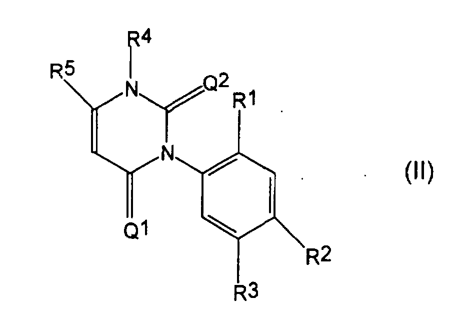 **caractérisé en ce que** :
Q¹ représente l'atome d'oxygène ou de soufre ;
Q² représente l'atome d'oxygène ou de soufre ;
R¹ représente l'atome de fluor ou de brome ;
R² représente le radical cyano ou le radical méthyle ;
R³ représente le radical amino ou nitro ;
R⁴ représente le radical amino, un radical alcoyle ayant 1 à 4 atomes de carbone, le cas échéant substitué par l'atome de fluor et/ou de chlore ou représente un radical cycloalcoyle ayant 3 à 6 atomes de carbone, et
R⁵ représente l'atome d'hydrogène ou un radical alcoyle ayant 1 à 4 atomes de carbone, le cas échéant substitué par l'atome de fluor et/ou de chlore.
